(19) Europäisches Patentamt
European Patent Office
Office européen des brevets

(11) Publication number: 0 314 042 A2

# (12) EUROPEAN PATENT APPLICATION

(21) Application number: 88117672.1

(22) Date of filing: 24.10.88

(51) Int. Cl.⁴: C08F 8/30 , C12N 9/96 , A61K 37/50

(30) Priority: 25.10.87 JP 268960/87
08.04.88 JP 87528/88

(43) Date of publication of application:
03.05.89 Bulletin 89/18

(84) Designated Contracting States:
AT BE CH DE ES FR GB IT LI NL SE

(71) Applicant: KURARAY CO., LTD.
1621 Sakazu
Kurashiki-City Okayama Prefecture 710(JP)

Applicant: Inoue, Masayasu
3-49-3, Ikeda
Kumamoto City Kumamoto(JP)

(72) Inventor: Inoue, Masayasu
49-3, Ikeda 3-chome
Kumamoto-City Kumamoto(JP)
Inventor: Ebashi, Iwao
1-30, Shinyashiki 3-chome
Kumamoto-City Kumamoto(JP)
Inventor: Watanabe, Nobukazu
9-9, Kamidoori-machi
Kumamoto-City Kumamoto(JP)
Inventor: Asano, Ryuzo
992-7, Ikaruga Taishi-cho
Ibo-District Hyogo(JP)
Inventor: Nakahara, Fumio
7-16, Amakidai 4-chome
Kurashiki-City Okayama(JP)
Inventor: Mori, Fumio
332-3, Ojima
Kurashiki-City Okayama(JP)

(74) Representative: Vossius & Partner
Siebertstrasse 4 P.O. Box 86 07 67
D-8000 München 86(DE)

## (54) Novel copolymers and superoxide dismutase modified with said copolymers.

(57) Described are copolymers comprising the copolymer-constituting units characterized in the claims and having an average molecular weight of 400 to 20,000. Furthermore superoxide dismutase (SOD) derivatives are described which can be obtained by modification with the above copolymers and have a single chemical structure, retain most of the enzymatic activity of SOD and exhibit a much prolonged plasma half-life. Therefore, they are useful as medicinal chemicals.

EP 0 314 042 A2

# NOVEL COPOLYMERS AND SUPEROXIDE DISMUTASE MODIFIED WITH SAID COPOLYMERS

This invention relates to novel copolymers and superoxide dismutase modified with said copolymers.

Superoxide dismutase (hereinafter abbreviated as SOD) has been known so far as an enzyme occurring widely in living organisms, such as animals, plants and microbes, and decomposing superoxide which is harmful to living organisms. Recently, attempts have been made to use isolated SOD as an anti-inflammatory agent [cf. Farumashia, 17, 411 (1981) and Current Therapeutic Research, 16, 706 (1974)].

Reportedly, the plasma half-life of intravenously administered SOD is 4 to 6 minutes. SOD is rapidly metabolized and excreted into urine. For prolonging the plasma half-life of SOD, attempts have been made to convert SOD to a giant molecule through modification with Ficoll, polyethylene glycol or albumin. Reportedly, however, in the Ficoll- or polyethylene glycol-modified-SOD, the enzymatic activity of SOD is markedly decreased and the albumin-modified SOD has antigenicity. It is known that inulin-modified SOD has a much prolonged plasma half-life but, at the same time, has a reduced enzymatic activity as compared with SOD [cf. Japanese Kokai Tokkyo Koho No. 58-32826].

One of the present inventors has previously found that an SOD derivative obtained by introducing a hydrophobic organic acid structure having a styrene-maleic acid skeleton onto the surface of the SOD molecule via an acid amide bond can reversibly bind to and dissociate from serum proteins (mainly albumin) in blood and, as a result, can increase the plasma half-life and organotropism of SOD [Abstracts of Papers presented at the 37th Tanpakushitsu Kozo Toronkai (Forum on Protein Structures), pages 61-64 (issued September 10, 1986); United States Patent Application Serial No. 049 349].

The above-mentioned Ficoll-, polyethylene glycol-, albumin-, and inulin-modified SOD species all have problems in their practical use for the reasons already mentioned above and/or on account of the decrease in tissue permeability due to the increase in molecular size.

Various modified SOD species so far known are all prepared by causing various macromolecular substances to bind to SOD via amino groups within the SOD molecule. Human-type SOD has 22 or 24 amino groups per molecule, while bovine-type SOD contains 20 amino groups per molecule. Since SOD has a number of amino groups, as just mentioned above, it is very difficult to specifically indicate the amino group which occurs within the SOD molecule and serves as the site of binding to a macromolecular substance used for the modification of SOD. It is preferable, however, that a medicinal chemical should be a compound identifiable by one single chemical structure. Under such circumstances, modified SOD species to be used for medicinal purposes should desirably be compounds having a chemical structure in which a macromolecular substance for modification is bound to SOD at a specific site within the SOD molecule.

It is an object of the invention to provide novel chemical modifiers capable of giving novel superoxide dismutase derivatives in which the enzymatic activity of SOD is substantially maintained and which have a significantly prolonged plasma half-life as compared with SOD and further have good transferability to organs, with a single chemical structure and without excessively increasing the molecular size.

Another object of the invention is to provide novel superoxide dismutase derivatives in which any of said chemical modifiers is bound to a specific site within the SOD molecule and which are moderate in molecular size and have a much prolonged plasma half-life as compared with SOD, with the enzymatic activity of SOD being substantially maintained therein.

A further object of the invention is to provide pharmaceutical uses of said superoxide dismutase derivatives as anti-inflammatory agents, protective agents against ischemic damages and agents for treating cerebral edema, among others.

A still further object of the invention is to provide a method of treating inflammations and/or ischemic diseases which comprises using said superoxide dismutase derivatives.

These objects as well as other objects and advantages of the present invention will become apparent to those skilled in the art from the following detailed description.

In accordance with the invention, the above objects can be accomplished by providing:

[I] A copolymer which comprises the polymer-constituting units (a) and (b):

(a) a group selected from the class consisting of groups of the formulas

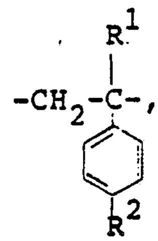, 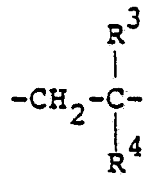, 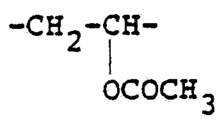 and 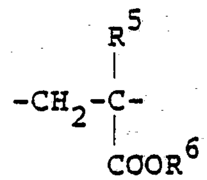

wherein $R^1$, $R^3$ and $R^5$ each independently is a hydrogen atom or a methyl group, $R^2$ is a hydrogen, chlorine or bromine atom or a methyl group, $R^4$ is a hydrogen atom, an alkyl group of 1 to 6 carbom atoms or a cycloalkyl group of 3 to 6 carbon atoms and $R^6$ is a methyl or ethyl group; and

(b) a group of the formula

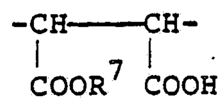

wherein $R^7$ is a hydrogen atom or the residue of an alkanol of 1 to 8 carbon atoms, of an ethylene glycol monoalkyl ether whose alkyl moiety contains 1 to 4 carbons atoms or of a glycerol dialkyl ether whose alkyl moieties each contains 1 to 4 carbon atoms as derived by removal of the hydroxyl group therefrom; and which further comprises:

(c) (i) internally, a group of the formula

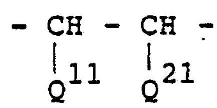

(I)

wherein one of $Q^{11}$ and $Q^{21}$ is a carboxyl group and the other is a group of the formula

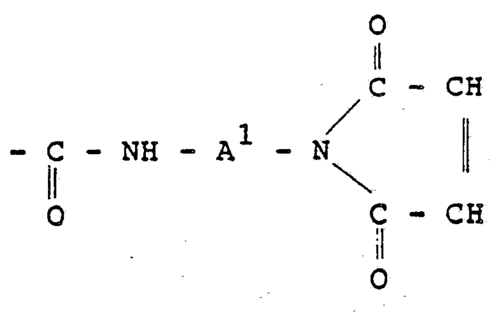

in which $A^1$ is a bivalent hydrocarbon residue which may optionally be interrupted by one or more groups each independently selected from the class consisting of an oxygen atom, a sulfur atom, a group of the formula $-N(R^8)-$ ($R^8$ being a hydrogen atom or an alkyl group of 1 to 4 carbom atoms) and a group of the formula -NH-CO-NH-; or

(ii) at one terminus, a group of the formula

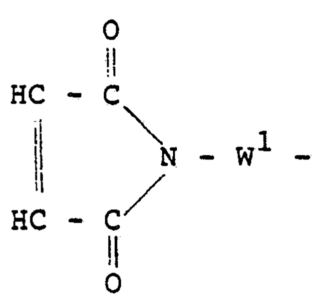

(II)

wherein $W^1$ is a bivalent organic group; said copolymer having an average molecular weight of 400 to 20,000 hereinafter, this copolymer is sometimes referred to generally as "copolymer" for short or sometimes to as "maleimide group-containing copolymer"];

[2] A superoxide dismutase derivative of the formula
$[SOD]\ [Z]_n$
wherein [SOD] is a univalent or divalent superoxide dismutase residue derived by hydrogen atom removal from one or two mercapto groups thereof, n is an integer of 1 or 2 corresponding to the valence of [SOD] and [Z] is a univalent group derived from a copolymer which comprises the polymer-constituting units (a) and (b):

(a)

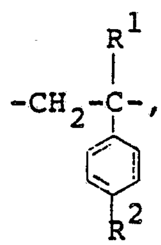 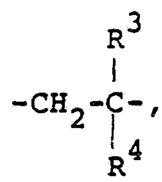 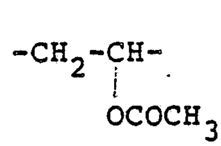 and 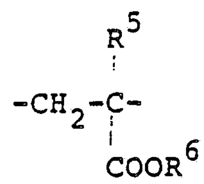

wherein $R^1$, $R^2$, $R^3$, $R^4$, $R^5$ and $R^6$ are as defined above;

(b) a group of the formula

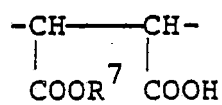

wherein $R^7$ is as defined above;
and which further comprises:

(c) (i) internally, a group of the formula

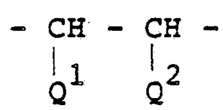 (I-A)

wherein one of $Q^1$ and $Q^2$ is a carboxyl group and the other is a group of the formula

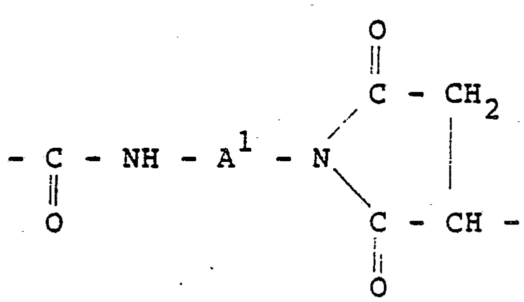 (II-A)

in which $A^1$ is as defined above, the succinimide ring carbon atom having a valence being bound to [SOD]; or

(ii) at one terminus, a group of the formula

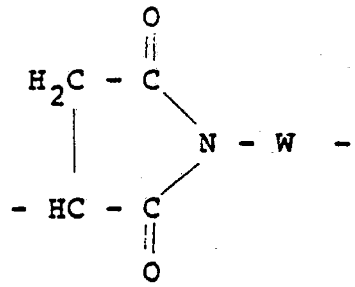

in which W is as defined above, the succinimide carbon atom having a valence being bound to [SOD]; said copolymer having an average molecular weight of 400 to 20,000 [said derivative is hereinafter referred to as "SOD derivative"];

[3] A pharmaceutical composition for the treatment of inflammation and/or ischemic disease which comprises an amount, effective for the treatment of inflammation and/or ischemic disease, of an SOD derivative as defined above and a pharmacologically acceptable diluent or carrier therefor; and

[4] A method of treating inflammation and/or ischemic disease which comprises administering an amount, effective for the treatment of inflammation and/or ischemic disease, of an SOD derivative as defined above to a patient with inflammation and/or ischemic disease.

Figs. 1 to 4 show infrared absorption spectra of the maleimide group-containing copolymers obtained in Examples 2, 3, 5 and 6, respectively.

Fig. 5 is a photograph showing the results of electrophoresis of the SOD species used in Example 9 [(a) in the figure], the reaction mixture obtained in Example 9 [(b)], the SOD- and SOD derivative-containing fraction obtained after gel filtration of said mixture [(c)], the SOD-containing fraction obtained from said fraction by affinity column chromatography [(d)] and the SOD derivative-containing fraction obtained by affinity column chromatography [(e)].

Fig. 6 shows ultraviolet absorption spectra of the SOD species used in Example 9 [(1) in the figure], the maleimide group-containing copolymer used in said example [(2)] and the SOD derivative obtained in said example [(3)].

Fig. 7 shows an infrared absorption spectrum of the SOD derivative obtained in Example 10.

Fig. 8 shows the results of electrophoresis of the SOD species used in Example 11 [(a)] in the figure], the reaction mixture obtained in said example [(b)], the SOD- and SOD derivative-containing fraction obtained from said mixture by gel filtration [(c)], the SOD-containing fraction obtained from said fraction by affinity column chromatography [(d)] and the SOD derivative-containing fraction obtained by affinity column chromatography [(e)].

Fig. 9 shows ultraviolet absorption spectra of the SOD species used in Example 14 [(1) in the figure], the maleimide group-containing copolymer used in the same Example [(2)] and the SOD derivative obtained therein [(3)].

Fig. 10 shows an infrared absorption spectrum of the SOD derivative obtained in Example 14.

Fig. 11 shows the time courses of the blood levels of SOD [(1)] in the figure] and an SOD derivative [(2)] as measured in Test Example 1.

Fig. 12 shows part of the electrocardiograms (lead II) obtained by continuous monitoring in Test Example 2 in an SOD-treated group [(A) in the figure] and an SOD derivative-treated group [(B)]. The electrocardiograms shown were taken before the second ischemia [(1) in the figure], 15 minutes after the second ischemia [(2)], 10 seconds [(3)], 30 seconds [(4)] and 3 minutes [(5)] after the second re-perfusion.

## Copolymers

Referring to formula (II), the bivalent organic group represented by W is not limited to any particular species provided that it serves to connect the valence of the nitrogen atom of the maleimido group with the copolymer comprising the constituent units (a) and (b). Typical examples of the group of the formula

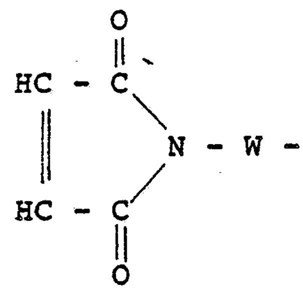

are groups of the formula

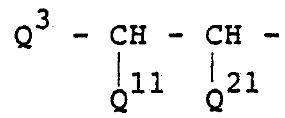

(II-1)

wherein $Q^{11}$ and $Q^{21}$ are as defined above and $Q^3$ is a univalent organic group, groups of the formula

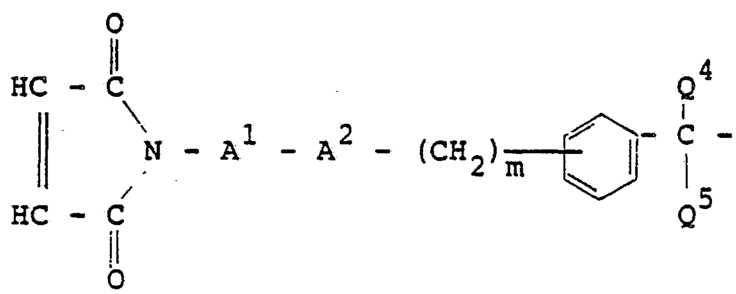

(II-2)

wherein $A^1$ is as defined above, $A^2$ is a group of the formula
-CO-O-, -CO-NH-, -CO-NH-$CH_2$-, -NH-CO-O-, -NH-CO-NH-, -NH-CO-NH-$CH_2$-, -N=CH-, -NH-$CH_2$- or

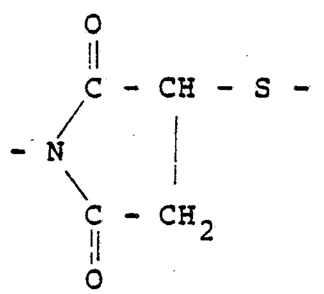

,

$Q^4$ and $Q^5$ each independently is a hydrogen atom or an alkyl group of 1 to 3 carbon atoms and m is an integer of 0 (zero) to 4, and groups of the formula

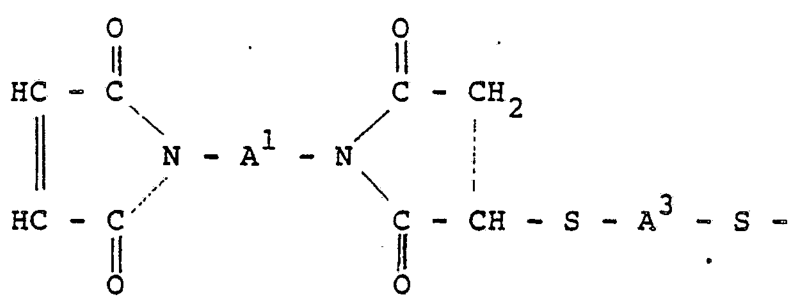

(II-3)

wherein $A^1$ is as defined above and $A^3$ is a bivalent hydrocarbon group, which may optionally be interrupted

by one or more oxygen and/or sulfur atoms.

These copolymers can be produced, for example, in the following manner:

[I] Copolymers comprising the constituent units (a) and (b) mentioned above and further an internal group of formula (I) or, at one terminus, a group of formula (II-1) and having an average molecular weight of 400 to 20,000 can be produced by reacting a compound of the formula

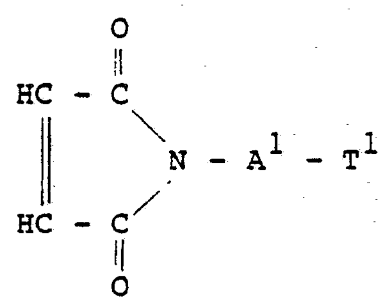

wherein $A^1$ is as defined above and $T^1$ is a group reactive with the maleic anhydride ring with a copolymer comprising the above-mentioned constituent units (a) and (b) and further 0.5 to 2 groups (per molecule) of the formula

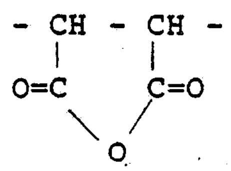

and having an average molecular weight of 400 to 20,000.

[II] Copolymers comprising the above-mentioned constituent units (a) and (b) and further, at one terminus, a group of formula (II) and having an average mnolecular weight of 400 to 20,000 can be produced by half-esterifying with an alkanol of 1 to 8 carbon atoms, an ethylene glycol monoalkyl ether whose alkyl moiety contains 1 to 4 carbon atoms or a glycerol dialkyl ether whose alkyl moieties each independently contains 1 to 4 carbon atoms and/or hydrolyzing a copolymer obtained by radical-copolymerizing (a′) a monomer selected from the class consisting of compounds of the formulas

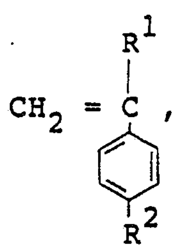 , 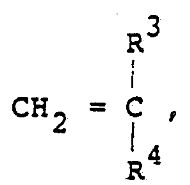 , 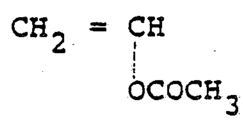 and 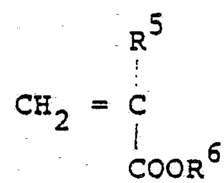

wherein $R^1$, $R^2$, $R^3$, $R^4$, $R^5$ and $R^6$ are as defined above, and

(b′) maleic anhydride in the presence of a polymeriza tion initiator having a functional group or a protected functional group and/or a chain transfer agent having a functional group or a protected functional group, and having, at one terminus, said functional group or protected functional group and an average molecular weight of 400 to 20,000; then eliminating the protective group from the terminal protected functional group if necessary, and reacting the thus-modified copolymer with a compound of the formula

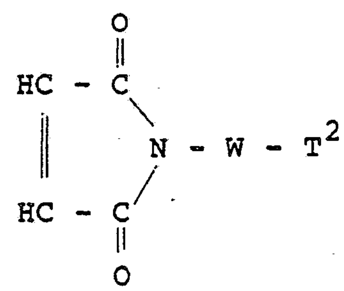

wherein W is as defined above and $T^2$ is a group reactive with the functional group of said modified copolymer.

The methods of producing the following three types of the maleimide group-containing copolymer are now described in further detail.

(1) Copolymers comprising the above-mentioned constituent units (a) and (b) and further, at one terminus, a group of formula (II-1) or an internal group of formula (I) [hereinafter referred to as "maleimide group-containing copolymers (1)];

(2) Copolymers comprising the above-mentioned constituent units (a) and (b) and further, at one terminus, a group of formula (II-2) [hereinafter referred to as "maleimide group-containing copolymers (2)]; and

(3) Copolymers comprising the above-mentioned constituent units (a) and (b) and further, at one terminus, a group of formula (II-3) [hereinafter referred to as "maleimide group-containing copolymers (3)].

1) Production of maleimide group-containing copolymers (1)

A copolymer of a monomer corresponding to the constituent unit (a) and maleic anhydride is half-esterified and/or hydrolyzed to an extent such that the product has, on an average, 0.5 to 2 (preferably about one) maleic anhydride rings per molecule. Then, the copolymer thus obtained is reacted with a compound of the formula

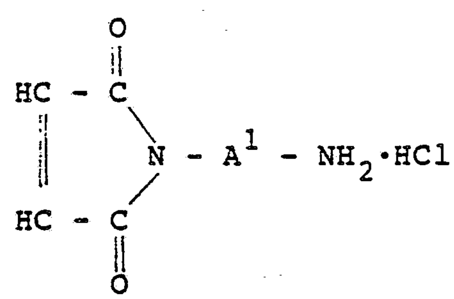 (III)

wherein $A^1$ is as defined above, in the manner of half amidation to give a maleimide-containing copolymer (1). This amidation reaction is generally carried out either in an aqueous solution of a salt such as sodium carbonate, sodium bicarbonate, sodium acetate, sodium phosphate or potassium phosphate, or in an organic solvent such as chloroform, 1,2-dichloroethane, dioxane, tetrahydrofuran, acetonitrile, dimethyl sulfoxide or N,N-dimethylformamide, if necessary in the presence of a base such as sodium bicarbonate, potassium carbonate, pyridine or triethylamine. When the reaction is conducted in an aqueous solution of such a salt, said reaction is preferably carried out at a pH of 9 or less with ice cooling or at a temperature up to room temperature, more preferably with ice cooling, in view of the stability of the maleimide ring. The reaction period is generally 1 hour to 1 day. The compound of formula (III) is used in an amount of about 0.5 to 10 moles per mole of the above-mentioned, maleic anhydride ring-containing copolymer. When the reaction is conducted in an organic solvent, said reaction is performed with ice cooling or at a temperature up to about 80 °C for about 1 to 10 hours. The compound of formula (III) is used in the same amount as mentioned above for the reaction in an aqueous salt solution. The base, if used, is used preferably in an amount of about 1 to 10 moles per mole of the compound of formula (III).

In the above-mentioned formula (II-1), $Q^3$ represents a univalent organic group, as mentioned above,

and this is a terminal or end group of the copolymer obtained by the radical polymerization. The end group may be a radical group generated by the radical initiator used as the catalyst, or a radical group generated as a result of chain transfer of the initiator-derived radical group to reaction solvent or another coexisting substance. Preferred as the univalent organic group $Q^3$ is a radical group resulting from chain transfer to a solvent, for example an aralkyl group, such as benzyl, α-methylbenzyl or α,α-dimethylbenzyl, which group may be substituted, on its nucleus, by one or more substituents selected from among halogen atoms such as chlorine and bromine atoms, lower alkoxy groups such as methoxy and propoxy, methyl, methoxymethyl, dimethoxymethyl, acetoxymethyl, acetylthio, acetylthiomethyl, methoxycarbonyl and so on.

2) Production of maleimide group-containing copolymers (2)

The monomer mentioned above under (a′) and maleic anhydride are radical-copolymerized in the presence of a compound of the formula

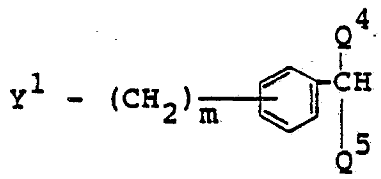

(IV)

wherein $Q^4$, $Q^5$ and m are as defined above, $Y^1$ is a group of the formula $-O-CO-R^9$, $-S-CO-R^{10}$, $-CH(OR^{11})_2$, $-NO_2$ or -CN in which $R^9$, $R^{10}$ and $R^{11}$ each independently is an alkyl group of 1 to 4 carbon atoms, which compound is used as a chain transfer agent and reaction solvent, if necessary in the presence of an organic solvent such as octane, benzene or acetone, to give a copolymer having, at one terminus, a group of the formula

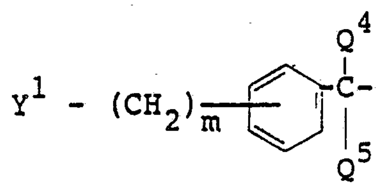

(IV')

wherein $Y^1$, $Q^4$, $Q^5$ and m are as defined above. Usable as the compound of formula (IV) are, for example, p-ethylbenzyl acetate, p-isopropylbenzyl acetate, S-p-isopropylphenyl thioacetate, S-p-isopropylbenzyl thioacetate, p-isopropylbenzaldehyde dimethylacetal, p-isopropylbenzaldehyde dibutyl acetal, p-nitrocumene and p-cyanocumene. The thus-obtained copolymer is half-esterified and/or hydrolyzed by a conventional method to cause the maleic anhydride ring occurring in said copolymer to disappear, and that group which occurs at one terminus of the copolymer and is represented by $Y^1$ occurring in the above-mentioned group of formula (IV′) is then converted to a reactive functional group, $Y^2$, to give a copolymner [hereinafter, "copolymer (IV″)"] having, at one terminus, a group of the formula

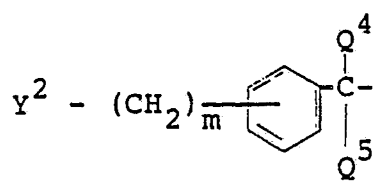

(IV")

wherein $Q^4$, $Q^5$ and m are as defined above and $Y^2$ is a hydroxyl, mercapto, formyl, amino or aminomethyl group. Among the groups represented by $Y^1$, the groups of the formula $-O-CO-R^9$, $-S-CO-R^{10}$ or $-CH(OR^{11})_2$ are hydrolyzed in the conventional manner to give a hydroxyl, mercapto or formyl group, respectively, whereas the nitro or cyano group is converted to an amino or aminomethyl group, respectively.
A group of the formula

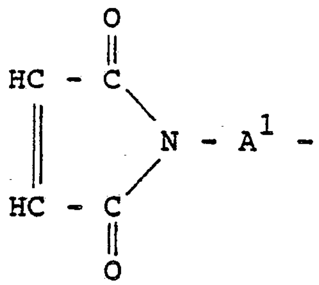

wherein $A^1$ is as defined above, is introduced into the copolymer (IV″) via the coupling group $A^2$ by utilizing the functional group $Y^2$ to give a maleimide group-containing copolymer (2). The reactions to be used for the formation of the coupling group $A^2$ are detailedly described in the following.

① When, in the copolymer (IV″), $Y^2$ is a hydroxyl, amino or aminomethyl group, the copolymer (IV″) is reacted with a reactive derivative of a carboxylic acid of the formula

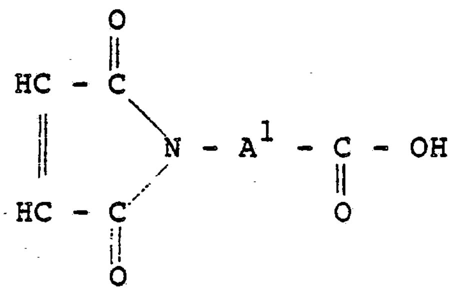

(V)

wherein $A^1$ is as defined above, or with an isocyanate compound of the formula

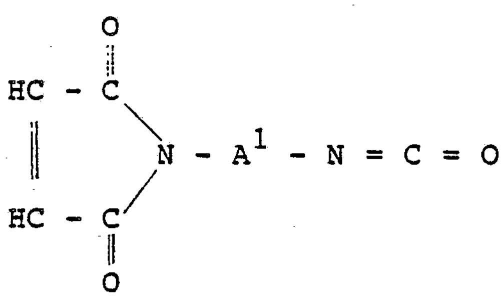

(VI)

wherein $A^1$ is as defined above, to give a maleimide group-containing copolymer (2). The reactive derivative of the carboxylic acid of formula (V) includes, among others, the acid chloride, acid anhydride, N-hydroxysuccinimide ester, acylimidazole and 8-hydroxyquinoline ester. The reaction of such reactive derivative of carboxylic acid and the copolymer (IV″) is carried out in the conventional manner in water or an organic solvent, such as chloroform, N,N-dimethylformamide, tetrahydrofuran, acetone, benzene or acetonitrile, if necessary in the presence of an auxiliary agent, such as pyridine, triethylamine or sodium bicarbonate. This reaction gives a maleimide group-containing copolymer (2) in which $A^2$ is a group of the formula $-CO-O-$, $-CO-NH-$ or $-CO-NH-CH_2-$. The reaction of the isocyanate of formula (VI) and the copolymer (IV″) is carried out in the conventional manner in an organic solvent, such as chloroform, tetrahydrofuran, acetone, dimethyl sulfoxide or N,N-dimethylformamide, at room temperature or with heating up to the boiling point of the solvent used. This reaction gives a maleimide group-containing copolymer (2) in which $A^2$ is a group of the formula $-NH-CO-O-$, $-NH-CO-NH-$ or $-NH-CO-NH-CH_2-$.

② When, in the copolymer (IV″), $Y^2$ is a formyl group, the copolymer (IV″) is reacted with a compound of formula (III) to give a maleimide group-containing copolymer (2) in which $A^2$ is $-N=CH-$. The reaction is carried out in the conventional manner under Schiff base formation conditions, namely under mild dehydrating conditions, generally in an organic solvent, such as chloroform, tetrahydrofuran or acetonitrile. When this reaction is conducted in the presence of, for example, sodium cyanoborohydride, the product is a maleimide group-containing copolymer (2) in which $A^2$ is a group of the formula $-NH-CH_2-$. The same product [copolymer (2); A = $-NH-CH_2-$] can also be obtained by reducing the maleimide group-containing

copolymer (2) obtained by the Schiff base formation reaction in which $A^2$ is a group of the formula -N=CH-, for example with sodium cyanoborohydride.

③ When, in the copolymer (IV″), $Y^2$ is a mercapto group, the copolymer (IV″) is reacted with a bismaleimide compound [hereinafter, "bismaleimide compound (VII)] of the formula

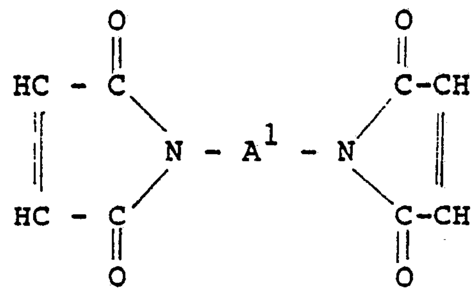

(VII)

wherein $A^1$ is as defined above, to give a maleimide group-containing copolymer (2) in which $A^2$ is a group of the formula

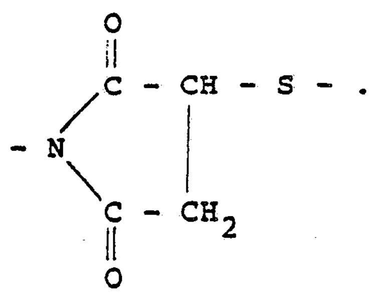

The reaction is generally carried out in a solvent, such as water, chloroform, tetrahydrofuran, ethanol, acetone or acetonitrile, if necessary in the presence of an auxiliary agent, such as pyridine, triethylamine, sodium bicarbonate or ammonium bicarbonate. Chloroform and acetonitrile are preferred solvents from the viewpoints of the solubilities of copolymer (IV″) and bismaleimide compound (VII) and the stabilities of maleimide ring and mercapto group, among others. It is preferable to use the bismaleimide compound (VII) in excess relative to the copolymer (IV″), generally in an amount of 1.5 to 10 moles, more preferably 3 to 5 moles, per mole of the copolymer (IV″). The reaction is carried out with ice cooling or at a temperature up to room temperature for about 1 to 10 hours.

3) Production of maleimide group-containing copolymers (3)

A copolymer [hereinafter, "copolymer (IX")"] obtained by the method mentioned later herein and having, at one terminus, a group of the formula
HS-$A^3$-S- (IX″)
wherein $A^3$ is as defined above, is reacted with a bismaleimide compound (VII) to give a maleimide group-containing copolymer (3). The reaction is carried out in the same manner as mentioned above under 2)-③.

The means to be used for the production of mercapto group-containing copolymers are now described in further detail. Copolymers comprising the above-mentioned constituent units (a) and (b) and further, at one terminus a group of formula SH-W′- in which W′ is a bivalent organic group and having an average molecular weight of 400 to 20,000 can be produced by half-esterifying and/or hydrolyzing in the same manner as mentioned above a copolymer obtained by radical-copolymerizing a monomer mentioned above under (a′) and the above-mentioned monomer (b′) in the presence of a chain transfer agent, which has a protected mercapto group, and having, at one terminus, said protected mercapto group and an average molecular weight of 400 to 20,000; then eliminating the protective group from said protected mercapto group. As the protected mercapto group-containing chain transfer agent to be used in the radical copolymerization, there may be mentioned compounds of the formula

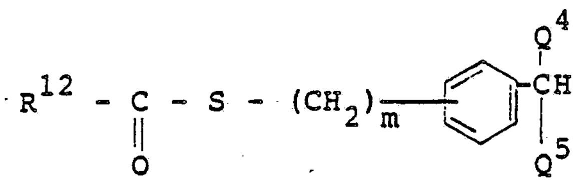

(VIII)

or

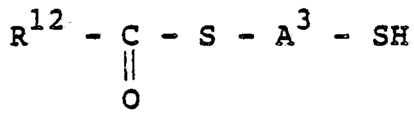

(IX)

wherein $R^{12}$ is an alkyl group of 1 to 3 carbon atoms and $Q^4$, $Q^5$, $A^3$ and m are as defined above.

Examples of the compound of formula (VIII) are esters of p-ethylbenzylmercaptan, p-isopropylbenzylmercaptan, p-ethylthiophenol, p-isopropylthiophenol, o-isopropylthiophenol and the like with acetic acid, propionic acid, butyric acid and the like. As the compound of formula (IX), there may be mentioned, for example, half esters of 1,2-ethanedithiol, 1,3-propanedithiol, 1,4-butanedithiol, 2-mercaptoethyl sulfide, di(2-mercaptoethyl) ether, 2,2′-dithiobisethanethiol, 1,2-benzenedithiol, 1,3-benzenedithiol, 3,4-toluenedithiol, 1,3-bis(mercaptomethyl)benzene, 1,4-bis(mercaptomethyl)benzene and the like with acetic acid, propionic acid, butyric acid and the like.

The above-mentioned chain transfer agent may also serve as the polymerization solvent. If necessary, however, an organic solvent, such as octane, benzene, acetone, ethylbenzene or methyl ethyl ketone, may be used additionally. When conducted in the presence of such chain transfer agent and, if necessary, of an organic solvent, the radical polymerization gives a copolymer having, at one terminus, a group of the formula

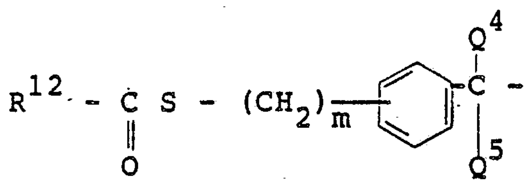

(VIII')

or

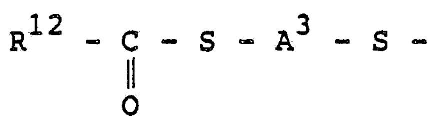

(IX')

wherein $R^{12}$, $Q^4$, $Q^5$, $A^3$ and m are as defined above. The maleic anhydride ring of the copolymer thus obtained is half-esterified and/or hydrolyzed and, furthermore, the terminal thio ester moiety is hydrolyzed to give a copolymer having, at one terminus, a group of the formula

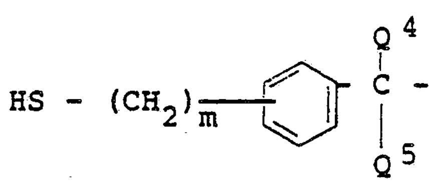

(VIII")

or

$HS - A^3 - S -$ (IX″)

wherein $Q^4$, $Q^5$, $A^3$ and m are as defined above [said copolymer being hereinafter referred to as "copolymer (VIII″) or (IX″)" according to the terminal group thereof].

The hydrolysis of the terminal thioester moiety can be conducted under mild conditions. Thus, for instance, the hydrolysis is carried out in an aqueous medium in the presence of sodium phosphate, potassium carbonate, sodium carbonate, sodium bicarbonate, sodium hydroxide, potassium hydroxide or

the like with ice cooling or at a temperature up to room temperature for several minutes to about 10 hours.

The procedures to be used for the isolation and purification of the copolymers from the reaction mixtures obtained in the above manner are now described. When the reaction is conducted in an aqueous medium, the reaction mixture obtained is directly subjected to gel filtration, and the desired eluate fraction is collected and lyophilized. When the reaction is conducted in an organic solvent, the organic solvent is removed from the reaction mixture obtained if such removal is deemed necessary, the residue is subjected to gel filtration in the same manner as above, and the desired eluate fraction is collected and lyophilized or, alternatively, the reaction mixture is concentrated as necessary or the organic solvent is removed therefrom as necessary, the remaining reaction mixture is subjected to preparative liquid chromatography using tetrahydrofuran, chloroform, acetone or the like as the developing solvent, and the desired eluate fraction is collected and lyophilized.

The methods of producing copolymers from a monomer corresponding to the above-mentioned constituent unit (a) and maleic anhydride and of producing half-esterified and/or hydrolyzed copolymers therefrom are now described in the following.

The radical copolymerization of a monomer corresponding to the constituent unit (a) and maleic anhydride is carried out in the presence of a catalyst such as dicumyl peroxide, azobisisobutyronitrile, benzoyl peroxide or t-butyl perbenzoate. The catalyst is used in an amount of about 1 to 10% by weight based on the monomer corresponding to the constituent unit (a). When a compound of formula (IV), (VIII) or (IX) is used as a chain transfer agent in carrying out the radical copolymerization reaction, said compound may also serve as a reaction solvent, as already mentioned above. If necessary, however, some other organic solvent may be used additionally. The copolymers obtained in this case are mostly used for the production of the above-mentioned maleimide group-containing copolymers (2) and (3), mercapto group-containing copolymers and active disulfide group-containing copolymers. When the radical copolymerization is effected in the absence of a compound of formula (IV), (VIII) or (IX), cumene, cymene, toluene, ethylbenzene, acetone, methyl ethyl keton, tetrahydrofuran, diethylene glycol dimethyl ether or the like are used each as a reaction solvent. Among these solvents, cumene, cymene, toluene and ethylbenzene are preferred. In this case, a chain transfer agent, such as benzyl alcohol, dodecylmercaptan or benzaldehyde, may be present in the reaction system. The copolymers obtained are used for the production of the above-mentioned maleimide group-containing copolymers (1). In either case, maleic anhydride is used in an amount of about 1 to 5 moles, preferably about 2 moles, per mole of the monomer corresponding to constituent unit (a). The total amount of the monomer corresponding to constituent unit (a) and maleic anhydride in the solvent is adjusted to about 5 to 40% by weight, preferably about 10 to 30% by weight. The reaction is carried out at a temperature within the range of about 40° to 180°C, preferably about 110° to 160°C. This radical copolymerization reaction is preferably carried out by slowly adding the monomer corresponding to constituent unit (a), together with the catalyst, to a solution of maleic anhydride in a solvent at the above temperature. The copolymers thus obtained by radical copolymerization are, either with or without fractionation for narrowing the molecular weight distribution, subjected to the subsequent reaction for maleic anhydride ring half-esterification and/or hydrolysis.

As the ester moiety obtained by maleic anhydride ring half-esterification of the copolymers of a monomer corresponding to constituent unit (a) and maleic anhydride, there may be mentioned the methyl ester, ethyl ester, propyl ester, n-butyl ester, n-amyl ester, isoamyl ester, n-hexyl ester, n-octyl ester, methoxyethyl ester, ethoxyethyl ester, propoxyethyl ester, 2-butoxyethyl ester, 1,3-dimethoxy-2-propyl ester, 2,3-dimethoxy-1-propyl ester, 1,3-diethoxy-2-propyl ester, 2-ethoxy-3-methoxy-1-propyl ester, 1,3-dipropoxy-2-propyl ester, 1,3-dibutoxy-2-propyl ester and benzyl ester, among others. Such half-esterified copolymers can be obtained by reacting the copolymers of a monomer corresponding to constituent unit (a) and maleic anhydride with a corresponding alcohol at a temperature within the range of about 50° to 120°C, if necessary in the presence of a catalyst such as lithium acetate. When used in excess, the corresponding alcohol can serve also as a solvent. Dioxane, tetrahydrofuran and the like can also be used each as the solvent.

The maleic anhydride ring hydrolysis of the copolymers of a monomer corresponding to constituent unit (a) and maleic anhydride is carried out in the conventional manner by using an aqueous solution of a base such as sodium hydroxide, potassium hydroxide or sodium bicarbonate. The desired copolymers can be isolated and purified from the reaction mixtures in the conventional manner by gel filtration or acid precipitation.

The monomer corresponding to constituent unit (a) includes, among others, styrene, p-chlorostyrene, p-bromostyrene, α-methylstyrene; ethylene, propylene, α-butylene,, isobutylene, 1-pentene, 2-methyl-1-butene, vinylcyclohexane, 1-hexene, 1-heptene; vinyl acetate; methyl (meth)acrylate and ethyl (meth)-acrylate.

It is preferable that, in the copolymers according to the invention, the mole ratio between the constituent (a) and constituent (b), namely the constituent mole ratio "(a)/(b)", is substantially within the range of about 1 to 1.3. In usual cases, said ratio is equal to 1. It is difficult to obtain copolymers having a constituent mole ratio (a)/(b) less than 1 by copolymerization of a monomer corresponding to constituent unit (a) and maleic anhydride. When the constituent mole ratio is greater than 1.3, the copolymers in which the constituent unit (b) is in the half-esterified form unfavorably show poor solubility in an aqueous salt solution in which said copolymers are to be reacted with SOD in the manner mentioned later herein.

The copolymers according to the invention have a weight average molecular weight within the range of 400 to 20,000. From the viewpoint of the transfer to affected sites of the SOD derivatives obtained by using the copolymers according to the invention as chemical modifiers for SOD, as mentioned later herein, however, the copolymers should preferably have a weight average molecular weight not exceeding 3,000. The molecular weight distribution of the copolymers is not critical. However, those copolymers for which the ratio between weight average molecular weight and number average molecular weight is within the range of about 1.02 to 1.3 can give SOD derivatives which are preferred as active ingredients of drugs or pharmaceutical compositions.

## SOD derivatives

Typical examples of the group of the formula

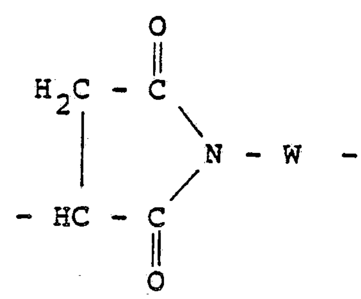

which occurs in the SOD derivatives according to the invention are groups of the formula

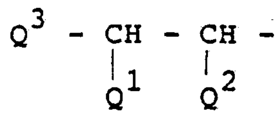

(II-A-1)

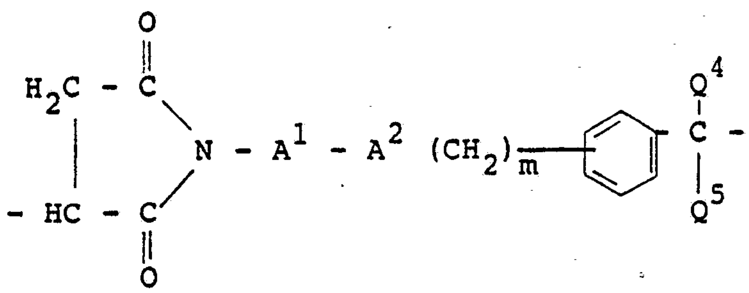

(II-A-2) or

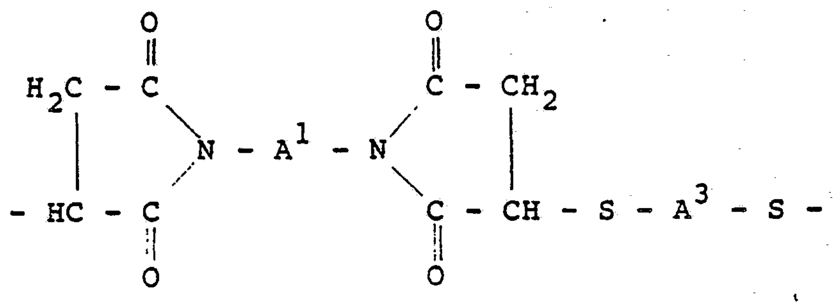

(II-A-3)

In the above formulas, one of $Q^1$ and $Q^2$ is a carboxyl group and the other is a group of the formula

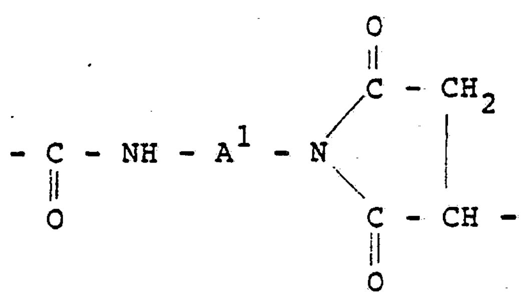

and $Q^3$, $Q^4$, $Q^5$, $A^1$, $A^2$, $A^3$ and m are as defined above.

The following description illustrates the production of SOD derivatives by using the maleimide group-containing copolymers according to the invention.

The SOD derivatives can be produced by reacting, in an aqueous solution having a pH of 6 to 10, SOD with a maleimide group-containing copolymer according to the invention.

The reaction between SOD and a maleimide group-containing copolymer is generally effected by adding the maleimide group-containing copolymer in the powder form or a solution prepared by dissolving said copolymer in an organic solvent, such as dimethyl sulfoxide, dioxane, acetone, tetrahydrofuran or ethyl alcohol, or in an aqueous solution of a salt, such as tris(hydroxymethyl)aminomethane hydrochloride, sodium carbonate, sodium bicarbonate, sodium acetate or sodium phosphate, to a solution prepared by dissolving SOD in the above-mentioned aqueous salt solution to an SOD concentration of 0.5 to 100 mg/ml, preferably 1 to 20 mg/ml. During the reaction, the pH of the solution should be maintained at 6 to 10. When the pH becomes lower than 6, the solubility of the maleimide group-containing copolymer decreases and the progress of the reaction becomes difficult. When the pH exceeds 10, the enzymatic activity of SOD itself may be affected depending on the length of the reaction period. The reaction should preferably be conducted at a pH lower than 9, since, at a pH of 9 or higher, the maleimide ring may be readily cleaved. As for the reaction temperature, room temperature or a temperature below room temperature is preferred and a temperature within the range of -5 °C to +5 °C is more preferred. The reaction period may vary depending on the reaction temperature and the manner of addition of the maleimide group-containing copolymer but generally is 10 minutes to 1 day. The maleimide group-containing copolymer is used in an

amount within the range of about 0.5 to 50 moles, per mole of SOD.

The thus-obtained reaction mixture contains the SOD derivative, unreacted SOD, unreacted maleimide group-containing copolymer and so on. The SOD derivative can be recovered in the solid form from such reaction mixture by subjecting said mixture to filtration, subjecting the filtrate to gel filtration, if necessary purifying the thus-obtained, SOD derivative-containing eluate fraction by hydrophobic column chromatography, affinity column chromatography, ion exchange columnn chromatography and/or the like, then concentrating said fraction by ultrafiltration and lyophilizing the concentrate.

In the above reaction, a mercapto group of SOD undergoes reaction with the maleimide group of the maleimide group-containing copolymer to give the desired SDO derivative.

Typical examples of the site of coupling between SOD and the copolymer are shown below in terms of structural formula. In the formulas shown below, [$Z^1$] and [$Z^2$] each indicates the principal chain of a copolymer.

(i) When the principal chain [Z] has, at one terminus, a group of formula (II-A-1) or, internally, a group of formula (I-A);

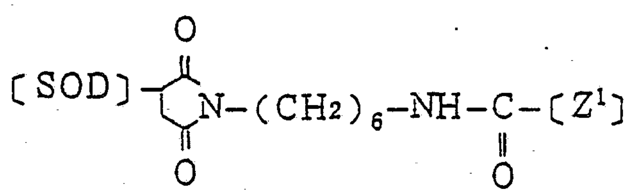

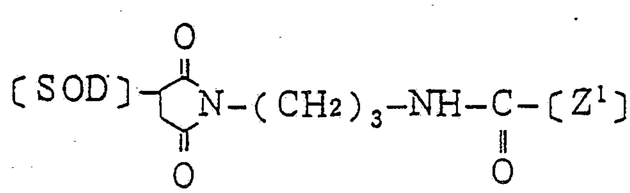

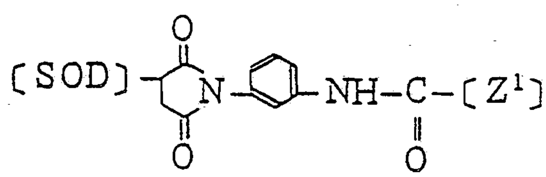

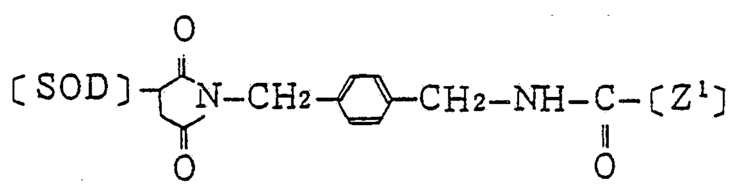

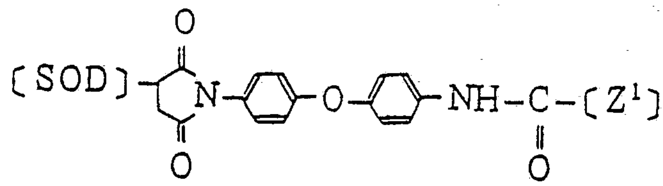

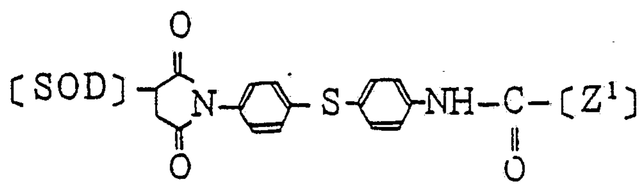

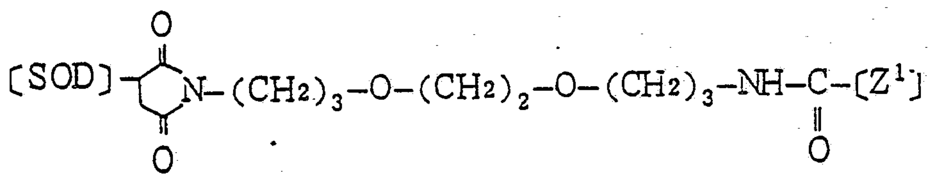

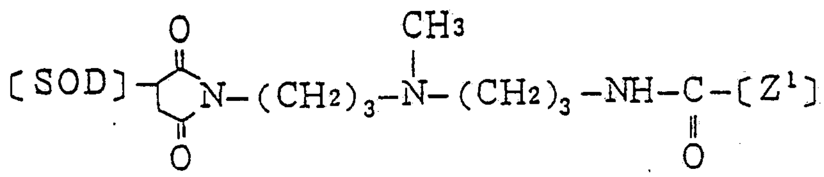

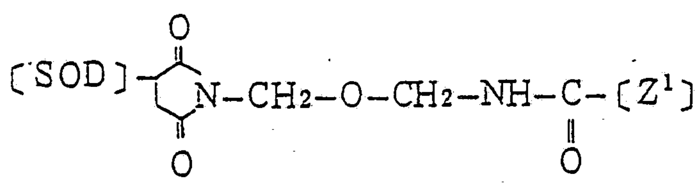

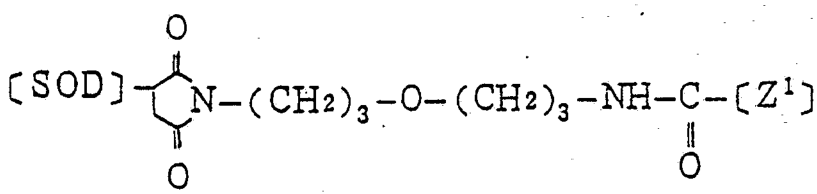

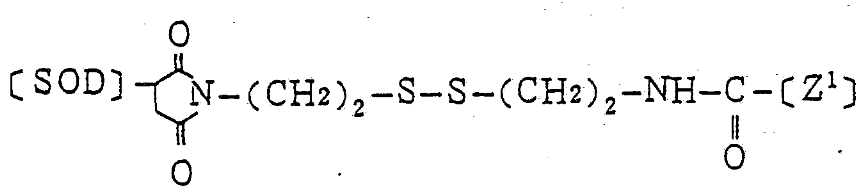

(ii) When [Z] has, at one terminus, a group of formula (II-A-2) or (II-A-3):

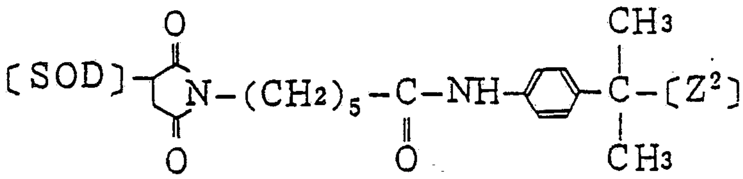

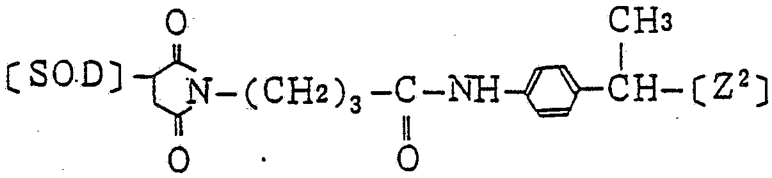

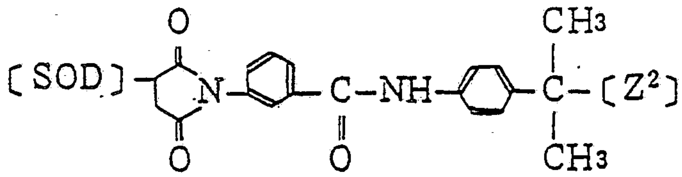

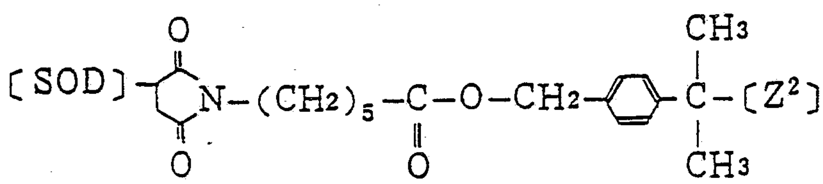

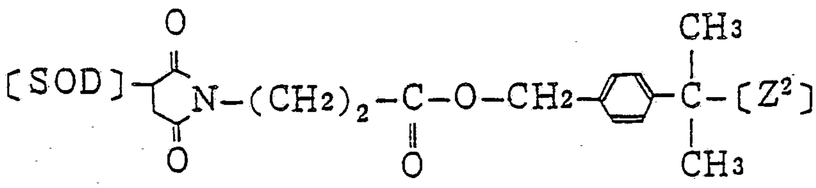

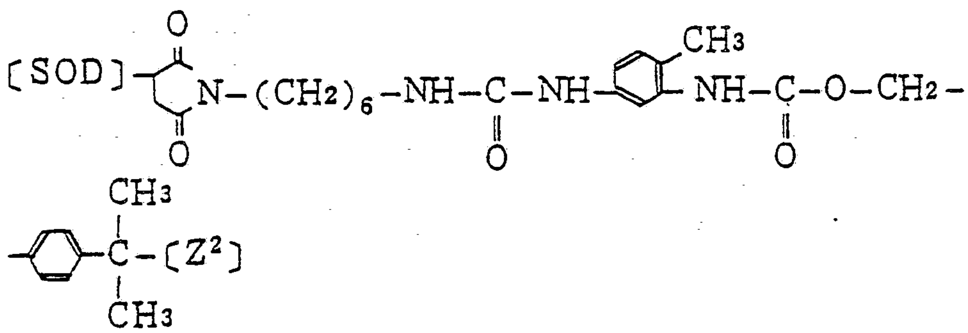

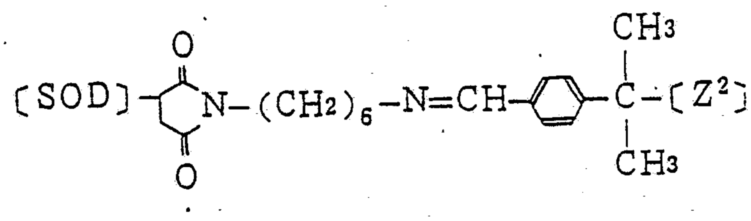

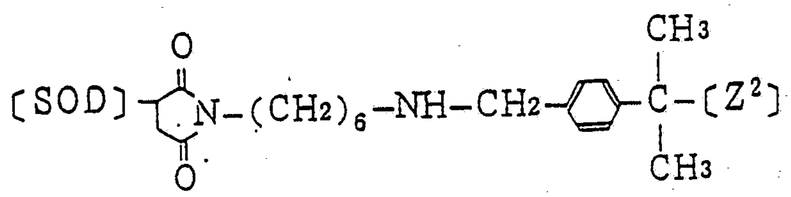

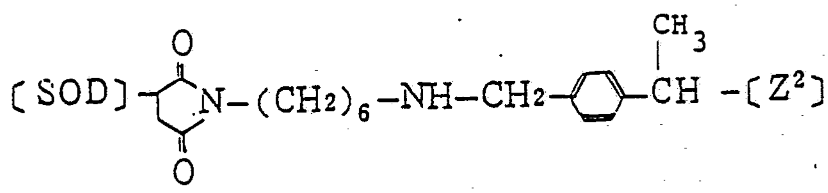

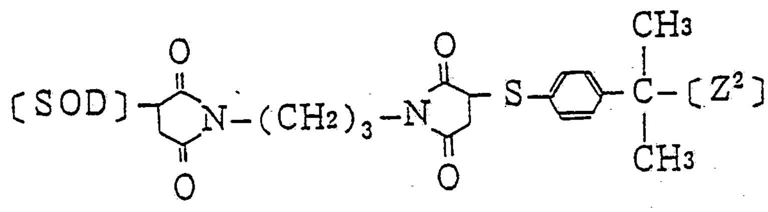

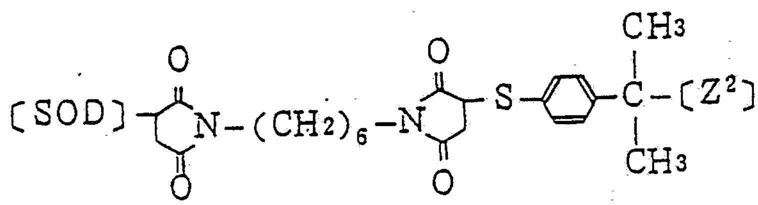

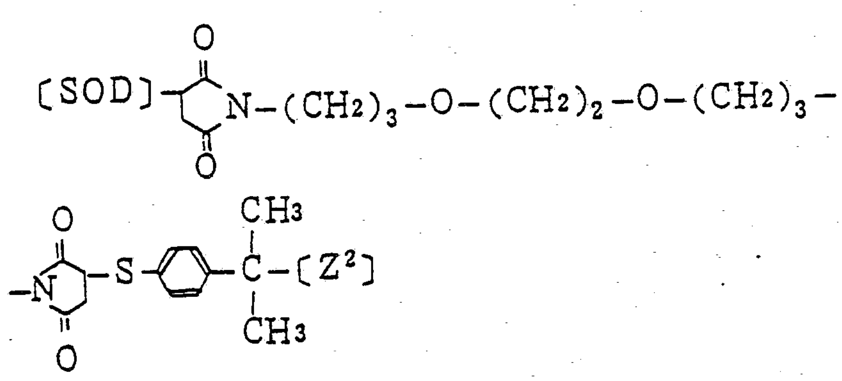

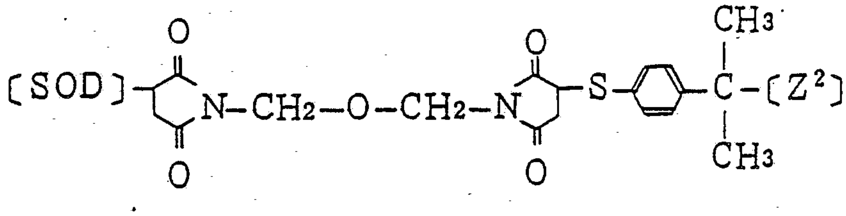

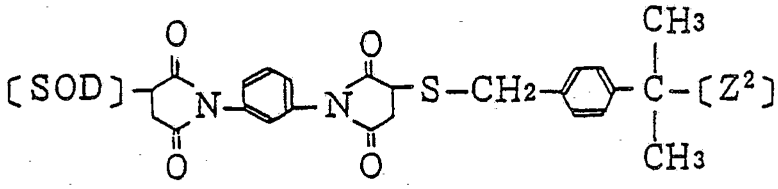

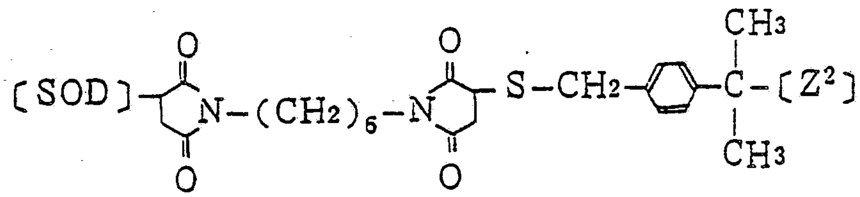

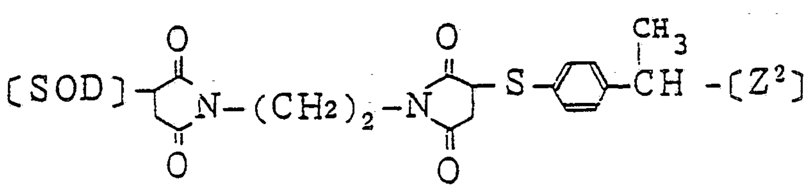

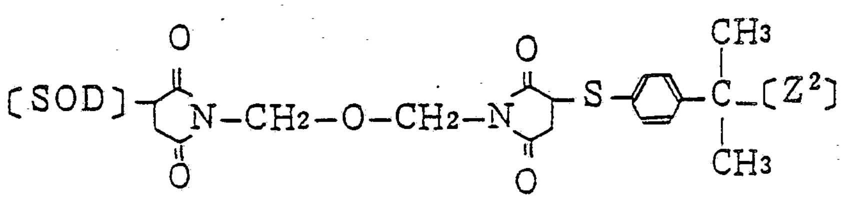

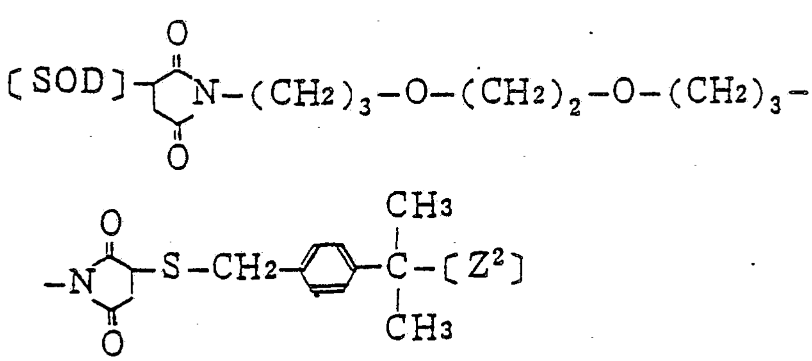

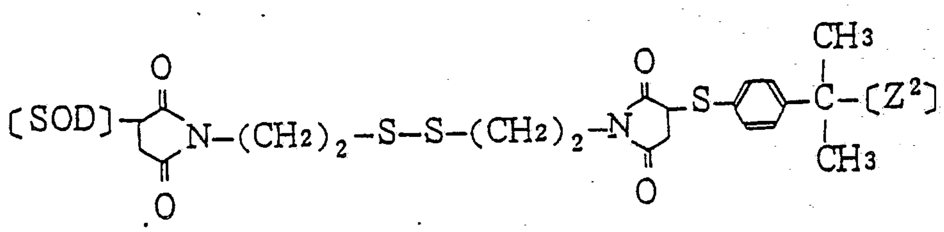

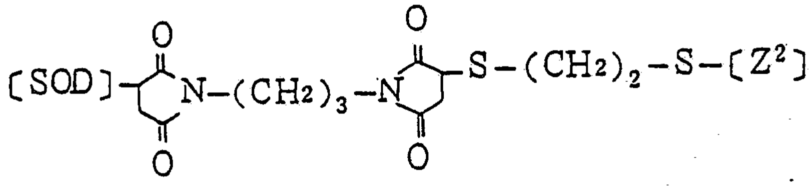

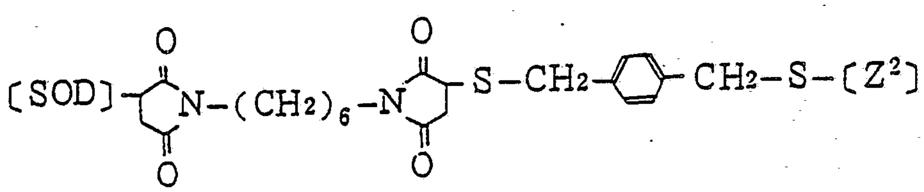

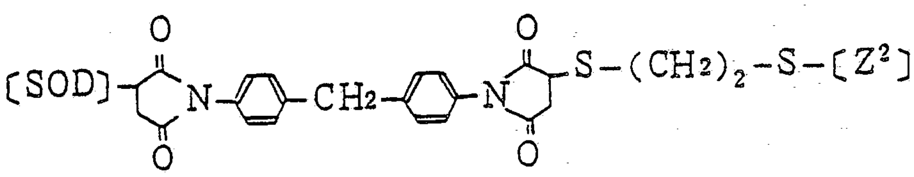

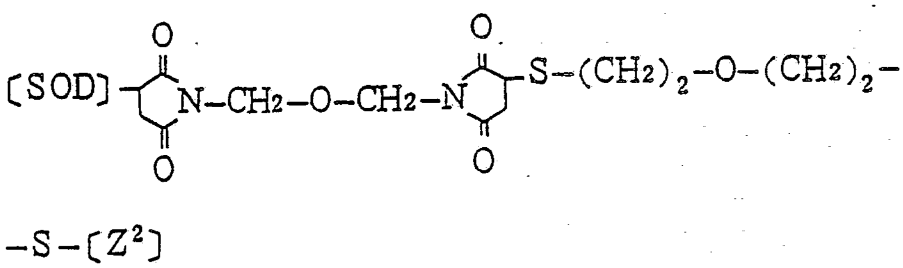

The copolymers according to the invention generally contain one maleimide per molecule. On the SOD side, human type SOD has two free, reactive mercapto groups. Therefore, depending on the reaction conditions mentioned above, an SOD derivative resulting from reaction of one or two molecules of copolymer with one molecule of human type SOD or a mixture of SOD derivatives resulting from reaction of one molecule and two molecules, respectively, of copolymer with one molecule of SOD is obtained. The use of such an SOD derivative mixture as an effective or active ingredient in pharmaceutical compositions will not cause any trouble. In view of the circumstances in the pharmaceutical field where compounds each having a single chemical structure are preferred as active ingredients in pharmaceutical compositions, however, it is preferable to subject the above SOD derivative mixture to column chromatography or some other adequate procedure and use the SOD derivatives thus separated each individually as an active ingredient in pharmaceutical compositions. In the reactions and subsequent treatment steps mentioned above, the SOD derivatives may form an alkali metal salt or ammonium salt because of their having carboxyl groups. However, the SOD derivatives in such salt form can be used as active ingredients in pharmaceutical compositions without any trouble.

Since the SOD derivatives are products of reaction of one molecule of SOD with at most two molecules of the copolymer, they are characterized, among others, in that the enzymatic activity of SOD is retained at a high level in them and that they have a significantly prolonged plasma half-life.

The copolymers according to the invention each has both a hydrophobic group and a hydrophilic group, hence a proper degree of hydrophobicity, and at the same time has highly polar carboxyl groups.

Therefore, the SOD derivatives comprising such copolymers bound to SOD can undergo reversible binding to serum proteins and biomembranes and, as a result, their plasma half-life is prolonged and their transferability to organs is improved. Among others, those SOD derivatives which have a styrene-derived group as the constituent unit (a) and those SOD derivatives which have a half-esterified group as the constituent unit (b) have higher hydrophobicity and are preferred from the viewpoint of the effects mentioned above.

## SOD

Usable SOD species may include SOD species contained in and obtained by a known method of extraction from animals (human, bovine, etc.), plants, microorga nisms and other living organisms as well as SOD species produced by genetic engineering techniques. The chemical structure (ligand metal, molecular weight, amino acid sequence, etc.) of SOD has been elucidated to a considerable extent, and SOD now includes three classes, namely Fe-SOD, Mn-SOD and Cu•Zn-SOD. The molecular weight of SOD is 30,000 to 80,000 depending on the SOD-containing living tissue. The amino acid sequence of SOD also differs to some extent from tissue to tissue. For detailed amino acid sequence data and so on, reference may be made to Yoshihiko Oyanagi: "Superoxide and Medicine", Kyoritsu Shuppan, published May 25, 1981; Journal of Biological Chemistry, 246, 2875-2880 (1971); ibid., 250, 6107-6112 (1975); Proceedings of the National Academy of Sciences of the U.S.A., 70, 3725-3729 (1973); and Archives of Biochemistry and Biophysics, 179, 243-256 (1977), among others. Human type Cu•Zn SOD has a molecular weight of 31,200 and two free, reactive mercapto groups. This human type SOD can be obtained, for example, by subjecting human blood in sequence to heat treatment, ion exchange and gel filtration or by using genetic engineering techniques.

## Pharmaceutical use of SOD derivatives

As evidenced by the results obtained in Test Example 2 to be mentioned later herein, the SOD derivatives have good anti-arrhythmic activity. In addition, the SOD derivatives have such pharmacological activities as anti-inflammatory, anti-ulcer, ischemic damage inhibitory and cerebral edema-preventing activities.

Toxicity testing has confirmed that the SOD derivatives have low toxicity.

The above results indicate that the SOD derivatives are effective in treating various diseases in which the active oxygen radical is involved and can be used, in particular, as anti-inflammatory agents, anti-ulcer agents, protective agents against ischemic damages, therapeutic agents for cerebral edema, and therapeutic agents for paraquat intoxication. The SOD derivatives are also useful as agents for alleviating adverse effects of anti-cancer agents which are due to the active oxygen radical. Furthermore, the SOD derivatives are useful as therapeutic agents for skin diseases, inclusive of burns, traumas and various kinds of dermatitis.

In addition to the above-mentioned pharmacological effects, the SOD derivatives efficiently retain the pharmacological effects known to be intrinsic to SOD [cf. Saishin Igaku, 39 (2), 339 (1984); Igaku to Yakugaku (Journal of Medicine and Pharmaceutical Science), 14 (1), 55 (1985); Jikken Igaku (Experimental Medicine), 4, (12), Special Issue "Free Radicals in vivo and Diseases":; and Fragrance Journal, No. 79, 89 (1986)]. The SOD derivatives are effective even against diseases in which the active oxygen radical is involved and against which SOD is not effective.

The dosage of the SOD derivatives varies with the kind of disease, severity of the disease, patient's tolerance, and other factors. However, the usual daily dosage for human adults is 0.1 to 500 mg, preferably 0.5 to 100 mg, either in a single dose or in a few divided doses. The SOD derivatives may be provided in various dosage forms suited for the respective routes of administration.

Thus the SOD derivatives can be formulated in making up pharmaceutical compositions by any of the established pharmaceutical procedures. Pharmaceutical compositions containing at least one SOD derivative as an active ingredient can be prepared by conventional means using pharmaceutically acceptable carriers, vehicles and other auxiliary substances which are commonly used in pharmaceutical practice.

When such pharmaceutical compositions are intended for oral administration, they are preferably provided in dosage forms suited for absorption from the gastro-intestinal tract. Tablets and capsules which

are unit dosage forms for oral administration may contain binders such as syrup, gum arabic, gelatin, sorbitol, gum tragacanth, polyvinylpyrrolidone, etc.; excipients such as lactose, corn starch, calcium phosphate, sorbitol, glycine, etc.; lubricants such as magnesium stearate, talc, polyethylene glycol, silica, etc.; disintegrators such as potato starch, etc.; pharmaceutically acceptable wetting agents such as sodium lauryl sulfate, etc.; and the like conventional ingredients. The tablets may be coated in the well-known manner. Liquid preparations for oral administration may be aqueous or oily suspensions, solutions, syrups, elixirs and so on, or may be lyophilisates which are extemporaneously reconstitued with water or other suitable vehicles. Such liquid preparations may contain usual additives inclusive of suspending agents such as sorbitol syrup, methylcellulose, glucose/sucrose syrup, gelatin, hydroxyethylcellulose, caboxymethylcellulose, aluminum stearate gel, hydrogenated edible oils and fats, etc; emulsifiers such as lecithin, sorbitan monooleate, gum arabic, etc; non-aqueous vehicles such as almond oil, fractionated coconut oil, oleaginous esters, propylene glycol, ethyl alcohol, etc; pre servatives such as methyl p-hydroxybenzoate, propyl p-hydroxybenzoate, sorbic acid, etc; and so forth.

For preparing injections, the SOD derivatives are dissolved in an appropriate solvent such as physiological saline, glucose solution for injection, etc. and the SOD derivative concentration is adjusted to 2 to 20 mg per 2 to 10 ml of solvent in the conventional manner to give injections for subcutaneous, intramuscular or intravenous administration. In preparing the above injections, pH-adjusting agents, buffers, stabilizers, preservatives, solubilizers and so forth may be added to the aqueous solution, if necessary.

The above-mentioned pharmaceutical compositions can contain the SOD derivative in a concentration selected according to the form thereof and other factors, generally in a concentration of about 0.01 to 50% by weight, preferably about 0.1 to 20% by weight.

## Advantages of the Invention

The present invention provides novel copolymers which can serve as novel chemical modifiers for SOD to give novel SOD derivatives each having a single chemical structure, retaining most of the enzymatic activity of SOD, showing a much prolonged plasma half-life as compared with unmodified SOD and showing good transferability to organs.

The copolymers are also useful as modifiers for proteins, enzymes other than SOD, or macromolecular compounds, which are reactive therewith.

The SOD derivatives obtained by binding said copolymers to SOD have the above-mentioned characteristic features. In addition, they can reversibly interact with serum proteins and this is advantageous in delivering SOD to affected sites. The SOD derivatives have excellent anti-arrhythmic activity. Furthermore, they have such pharmacological activities as anti-inflammatory, anti-ulcer, ischemic damage inhibitory and cerebral edema-preventing activities as well.

The following examples are further illustrative of the present invention but are by no means limitative of the invention.

## Example 1

### Synthesis of maleimide group-containing copolymer (1)

#### (a) Synthesis of N-(6-aminohexyl)maleimide hydrochloride

In 90 ml of dioxane was dissolved 23.20 g (0.20 mole) of 1,6-diaminohexane and a solution of 24.60 g 0.10 mole) of 2-(t-butoxycarbonylthio)-4,6-dimethylpyrimidine in 100 ml of dioxane was added dropwise thereto over a period of 3 hours. After completion of dropwise addition, the mixture was stirred overnight. The reaction mixture was filtered to separate the precipitate and the filtrate was concentrated to about 100 ml. This concentrate was diluted with 150 ml of water and the precipitated bis(N,N′-t-butoxycarbonyl)-1,6-diaminohexane was filtered off. The resulting filtrate was concentrated under reduced pressure to about half the initial volume. To the concentrate was added 40 g of sodium chloride and the mixture was extracted 4 times with 50 ml of ethyl acetate. The extracts were pooled and the ethyl acetate was distilled off under

reduced pressure. The residue was dissolved in 100 ml of water and the solution was adjusted to pH about 3 with 1N-hydrochloric acid. This aqueous solution was washed with ethyl acetate a few times and sodium chloride was added to the aqueous layer to precipitate N-t-butoxycarbonyl-1,6-diaminohexane hydrochloride. The yield of this compound was 15.10 g [yield based on 2-(t-butoxycarbonylthio)-4,6-dimethylpyridine: 60%).

To 75ml of chloroform were added 5.15 g (20 m moles) of N-t-butoxycarbonyl-1,6-diaminohexane hydrochloride, 1.95 g (20 m moles) of maleic anhydride and 2.03 g (20 m moles) of triethylamine and the reaction was carried out under reflux for 4 hours. After completion of the reaction, the solvent was distilled off under reduced pressure and 6.30 g of anhydrous sodium acetate and 65 ml of acetic anhydride were added to the residue. The reaction was then carried out at about 90 °C for 4 hours. The reaction mixture was cooled to room temperature and the precipitate was filtered off. From the filtrate, the solvent was distilled off under reduced pressure and the residue was subjected to silica gel column chromatography using a 2:8 (v/v) mixture of acetone and n-hexane as the eluent. The procedure gave 2.48 g of 6-(N-maleimide)-N′-t-butoxycarbonyl-1-aminohexane (yield based on N-t-butoxycarbonyl-1,6-diaminohexane hydrochloride: 41%).

To 16.8 ml of 4N-HCl in dioxane was added 2.36 g (8.0 m moles) of 6-(N-maleimido)-N′-t-butoxycarbonyl-1- aminohexane and the mixture was stirred at 15 °C for 1 hour. To the reaction mixture was added 120 ml of diethyl ether and the mixture was further stirred for 30 minutes. The resulting precipitate was recovered by filtration. The precipitate was recrystallized from chloroform-methanol to give 1.4 g of N-(6-aminohexyl)maleimide hydrochloride [yield based on 6-(N-maleimido)-N′-t-butoxycarbonyl-1-aminohexane: 76%].

m.p.: 156-158 °C

Elemental analysis (%):

| | | | | |
|---|---|---|---|---|
| Found: | C 51.36, | H 7.48, | N 11.92, | Cl 14.98 |
| Calcd.: | C 51.61, | H 7.36, | N 12.04, | Cl 15.23 |

(b) Synthesis of partially half-n-butyl-esterified styrene-maleic anhydride copolymer

To 3.7 ℓ of cumene was added 383.0 g (3.90 moles) of maleic anhydride and the mixture was refluxed with stirring. A mixed solution of 16.3 g (60 m moles) of dicumyl peroxide and 203.0 g (1.95 moles) of styrene in 1.7 ℓ of cumene was then added dropwise to the above refluxing mixture over a period of about 40 minutes. After completion of dropwise addition, the mixture was further stirred under heating for 1 hour. The resulting reaction mixture was allowed to stand overnight and after removal of the supernatant, the viscous product adherent to the vessel was dissolved in acetone and recovered. From this solution, the acetone was distilled off under reduced pressure to give 353.0 g of styrene-maleic anhydride copolymer.

In 3.0 ℓ of acetone was dissolved 120.0 g of the above styrene-maleic anhydride copolymer and, then, 4.9 ℓ of n-hexane was added dropwise to the solution over about 1 hour with constant stirring. This mixed acetone-n-hexane solution showing white turbidity was transferred to a different vessel, in which 9.7 ℓ of n-hexane was added dropwise under stirring over a period of about 1.5 hours. The syrupy product adherent to the vessel wall was dissolved in acetone and recovered. From this product, the acetone was distilled off under reduced pressure and the residue was dried in vacuo overnight at 70-80 °C, whereby 49.2 g of fractionally precipitated styrene-maleic anhydride copolymer was obtained.

In 1.0 ℓ of acetone was dissolved 40.0 g of the fractionally precipitated styrene-maleic anhydride copolymer and 3.8 kg of surface-silanated glass beads (average diameter 0.1 mm) were added to the solution. As the acetone was evaporated off, the fractionally precipitated styrene-maleic anhydride copolymer was deposited on the glass beads. A column measuring 80 mm in inside diameter and 80 cm long was packed with the above glass beads and 1.4 ℓ of a mixture of acetone and n-hexane (v/v = 24:76 at 25 °C). With the column temperature controlled at 25 °C, 1.6 ℓ of (i) acetone-n-hexane (24:76 v/v at 25 °C), 6.0 ℓ of (ii) acetone-n-hexane (37:63 v/v at 25 °C) and 3.0 ℓ of acetone were passed through the column from top to bottom. The fractions obtained with (ii) acetone-hexane (37:73 v/v) were pooled. From this eluate, the low-boiling fraction was distilled off under reduced pressure and the residue was dried in vacuo at 70-80 °C overnight, whereby 31.8 g of fractionated styrene-maleic anhydride copolymer was obtained. The weight average molecular weight ( $\overline{M}w$) and number average molecular weight ( $\overline{M}n$) of this copolymer were determined by gel permeation chromatography (briefly GPC; eluent: tetrahydrofuran;

standard; polystyrene; the same applies hereinafter). The values of $\overline{M}w$ and $\overline{M}n$ were 1340 and 1220, respectively, and the $\overline{M}w/\overline{M}n$ ratio was 1.10. The value of $\overline{M}n$ found by means of a vapor pressure-osmotic pressure meter was 1210. The maleic anhydride ring content of the above fractionated styrene-maleic anhydride copolymer, as determined by potentiometric titration, was 47.3 mole percent.

An ampule having a capacity of about 40 ml and equipped with a magnetic stirrer was charged with 10.00 g of the fractionated styrene-maleic anhydride copolymer, 2.20 g of n-butyl alcohol, 0.10 g of anhydrous lithium acetate and 20 ml of dioxane. After sealing, the contents of the ampule were heated with stirring at 90°C for 20 hours. A portion of the reaction mixture was sampled and the unreacted n-butyl alcohol in the reaction mixture was determined by gas chromatography using ethyl-cellosolve as the internal standard. From the reaction rate of n-butyl alcohol feed, the degree of half butyl esterification of maleic anhydride rings present in the copolymer was calculated. The degree so found was 62%. Then, the reaction mixture was diluted with 20 ml of dioxane and the dilution was added dropwise to 400 ml of n-hexane for re-precipitation. The resulting precipitate was recovered and dried in vacuo overnight at about 60°C, whereby 11.50 g of partially half-n-butyl-esterified styrene-maleic anhydride copolymer was obtained. The residual maleic anhydride ring content of this copolymer was determined from the intensities of absorption at 1780 $cm^{-1}$ and 700 $cm^{-1}$ in its infrared absorption spectrum (FT-IR, KBr disk; the same applies hereinafter). The residual content thus found was 30.3 mole % (an average of 1.8 maleic anhydride rings per molecule). As determined by GPC, the $\overline{M}$ and $\overline{M}n$ values of this product were 1530 and 1440, respectively, and the $\overline{M}w/\overline{M}n$ ratio was 1.06.

(c) Synthesis of a maleimide group-containing copolymer by reaction between N-(6-aminohexyl)maleimide hydrochloride and partially half-n-butyl-esterified st rene-maleic anhydride copolymer

To 8.0 ml of 0.8M sodium hydrogen carbonate-water was added 800 mg (0.5 m moles) of the partially half n-butyl-esterified styrene-maleic anhydride copolymer obtained in (b) above and the mixture was stirred at about 3°C for 3.5 hours, whereby the maleic anhydride ring content of the copolymer was decreased to about 1 per molecule on the average. To this reaction mixture was added 116 mg (0.5 m mole) of the N-(6-aminohexyl)maleimide hydrochloride prepared in (a) above and the mixture was reacted at about 3°C for 24 hours. The resulting reaction mixture was sampled and the amino group reaction rate was determined by the TNBS (sodium trinitrobenzenesulfonate) method. The reaction rate so found was 89%. This reaction mixture was filtered and the filtrate was subjected to gel permeation chromatography using Sephadex G-15, Pharmacia Fine Chemicals, as the support [column: K50/60 (Pharmacia Fine Chemicals); eluent: 5 mM ammonium bicarbonate-water: 5 mM ammonium carbonate-water (6:4, v/v; linear velocity: 11.0 cm/hr]. The fractions rich in the desired product were pooled and lyophilized to give 600 mg of maleimide group-containing copolymer whose physical data are given hereinbelow. Thus, this product was a mixture of a copolymer having, as constituent unit (a), a group of the formula

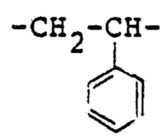

and, as constituent unit (b), a group of the formula

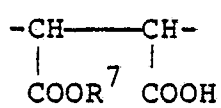

(wherein $R^7$ is n-butyl or hydrogen) and having, at one terminus, a group of the formula

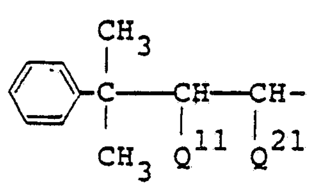

(wherein one of $Q^{11}$ and $Q^{21}$ is carboxyl, with the other being

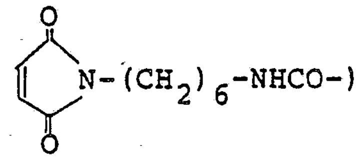

and a copolymer having, as constituent unit (a), a group of the formula

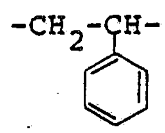

and, as constituent unit (b), a group of the formula

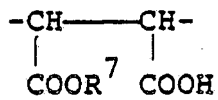

(wherein $R^7$ is n-butyl or hydrogen) and having, between said constituents, a group of the formula

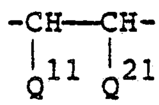

(wherein one of $Q^{11}$ and $Q^{21}$ is carboxyl, with the other being

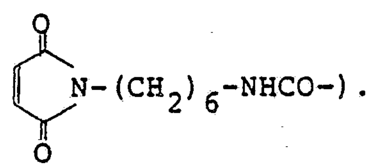

The average maleimide group content of this product was 0.7 per copolymer molecule. The maleimide group content was determined by the 4PDS (4,4′-dithiopyridine) method (the same applies to the following example).

Ultraviolet absorption spectrum: λmax 305 nm

Infrared absorption spectrum: 3440, 2960, 1730, 1710, 1570, 1180, 700 $cm^{-1}$

Elemental analysis (%, found): C 62.80, H 6.94, N 3.04

Molecular weight (GPC analysis): $\overline{M}w = 1810$, $\overline{M}n = 1700$, $\overline{M}w/\overline{M}n = 1.06$

Example 2

Synthesis of maleimide group-containing copolymer (1)

(a) Synthesis of partially hydrolyzed styrene-maleic anhydride copolymer

In 30.0 ml of dioxane was dissolved 10.0 g of a styrene-maleic anhydride copolymer obtained by the

procedure set forth in Example 1 (b) and after addition of 15.0 ml of water, the solution was refluxed with stirring for 24 hours. This reaction mixture was directly lyophilized to give 10.2 g of a partially hydrolyzed styrene-maleic anhydride copolymer having the under-mentioned physical properties. The maleic anhydride ring content of this product was determined by infrared absorption spectrophotometry in the same manner as Example 1 (b). The average content thus found was 1.0 per copolymer molecule.
Molecular weight (GPC analysis): $\overline{M}w = 1460$, $\overline{M}n = 1340$, $\overline{M}w/\overline{M}n = 1.09$

(b) Synthesis of a maleimide group-containing copolymer by reaction between N-(6-aminohexyl)maleimide hydrochloride and partially hydrolyzed styrene-maleic anhydride copolymer

To 8.0 ml of 0.8 M aqueous sodium hydrogen carbonate solution was added 116 mg (0.5 m mole) of the N-(6-aminohexyl)maleimide hydrochloride obtained by the procedure set forth in Example 1 (a) and while the mixture was stirred with ice-cooling, 800 mg (0.55 m mole) of the partially hydrolyzed styrene-maleic anhydride copolymer obtained in (a) above was gradually added and the reaction was conducted under stirring for 4 hours. The resulting reaction mixture was sampled and the amino group reaction rate was determined by the TNBS method. The reaction rate so found was 78%. The reaction mixture was then subjected to gel permeation chromatography in the same manner as Example 1 (c) and the eluate was lyophilized to give 560 mg of the desired maleimide group-containing copolymer. This product was a mixture of a copolymer having, as constituent unit (a), a group of the formula

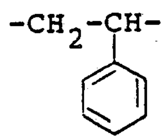

and, as constituent (b), a group of the formula

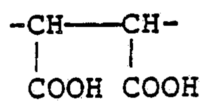 ,

and further having, as one terminus, a group of the formula

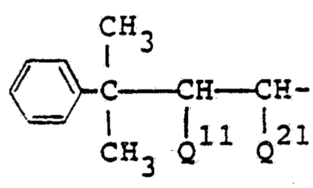

(wherein one of $Q^{11}$ and $Q^{21}$ is carboxyl, with the other being

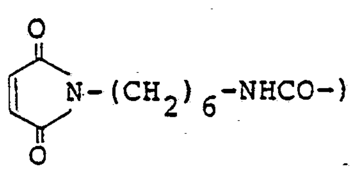

and a copolymer having, as constituent unit (a), a group of the formula

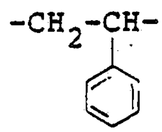

and, as constituent unit (b), a group of the formula

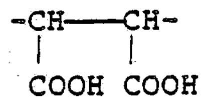

and further having, between said constituent units, a group of the formula

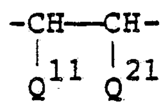

(wherein one of $Q^{11}$ and $Q^{21}$ is carboxyl, with other being

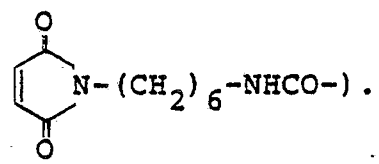

The average maleimide group content of this product was 0.6 per copolymer molecule. The infrared absorption spectrum of this copolymer is shown in Fig. 1.

Example 3

Synthesis of maleimide group-containing copolymer (2)

(a) Synthesis of 6-(N-maleimido)caproyl chloride

To 80 ml of chloroform were added 13.20 g (0.10 mole) of 6-amino-n-caproic acid and 9.80 g (0.10 mole) of maleic anhydride and the mixture was refluxed with stirring for 5 hours. From this reaction mixture, the chloroform was distilled off and 80 ml of acetic anhydride and 2.50 g of anhydrous sodium acetate were added. The mixture was stirred at 90 °C for 4 hours. This reaction mixture was cooled to room temperature and the resulting precipitate was filtered off. From the filtrate the solvent was distilled off, whereby a viscous liquid was obtained. This liquid was subjected to silica gel column chromatography using a 2:8 (v/v) mixture of acetone and n-hexane as the eluent. The procedure gave 5.40 g of 6-(N-maleimido)caproic acid having the following physical values.

m.p.: 84.0-85.0 °C

UV absorption spectrum: $\lambda$max 298 nm

IR absorption spectrum: 2940, 1705, 1406, 818, 700 $cm^{-1}$

To 5 ml of chloroform, stripped of ethanol by distillation, were added 253 mg (1.2 m moles) of 6-(N-maleimido)caproic acid, 714 mg (6.0 m moles) of thionyl chloride and 10 µl of pyridine and the mixture was stirred at room temperature for 5 hours. From the reaction mixture, the low-boiling fraction was distilled off to give 283 mg of 6-(N-maleimido)caproyl chloride.

(b) Synthesis of a styrene-maleic anhydride copolymer having a group of the formula

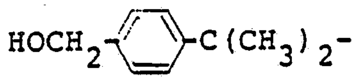

at one terminus and having been half-n-butyl-esterified

To 30 ml of p-isopropylbenzylacetic acid was added 4.30 g (44 m moles) of maleic anhydride and the mixture was stirred at 150° C to dissolve. To this solution, a mixed solution of 0.16 g (0.59 m mole) of dicumyl peroxide and 2.30 g (22 m moles) of styrene in 15 ml of p-isopropylbenzylacetic acid was added dropwise over a period of about 17 minutes. After completion of dropwise addition, the mixture was further stirred under heating for 15 minutes. This reaction mixture was cooled and added dropwise to 2 ℓ of hexane with stirring over about 30 minutes. The resulting precipitate was recovered by filtration and dried to give 8.10 g of a styrene-maleic anhydride copolymer having a group of the formula

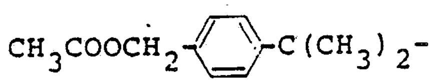

at one terminus.

In 160 ml of acetone was dissolved 8.00 g of the above styrene-maleic anhydride copolymer. Then, 240 ml of n-hexane was added dropwise to the solution under stirring over a period of about 20 minutes. The resulting acetone-n-hexane mixture showing white turbidity was transferred to another vessel, in which 400 ml of n-hexane was added dropwise over about 30 minutes, with constant stirring. The resulting syrupy product was dissolved in acetone and recovered. From this solution, the acetone was distilled off under reduced pressure and the residue was dried in vacuo at 70-80° C overnight, whereby 2.80 g of a fractionally separated styrene- maleic anhydride copolymer having a group of the formula

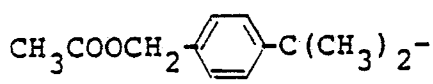

at one terminus was obtained. As determined by GPC analysis, the $\overline{M}w$ and $\overline{M}n$ values of this copolymer were 1460 and 1190, respectively, and the $\overline{M}w$/ $\overline{M}n$ ratio was 1.23.

To 20 ml of n-butyl alcohol were added 1.00 g of the fractionally precipitated styrene-maleic anhydride copolymer and 10 mg of anhydrous lithium acetate and the mixture was stirred at 95° C for 20 hours. Then, the n-butyl alcohol was distilled off under reduced pressure. To the residue was added 10 ml of dioxane and the mixture was lyophilized to give 1.20 g white powders of a styrene-maleic anhydride copolymer having a group of the formula

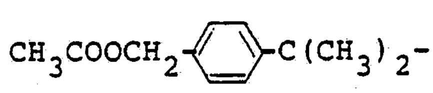

at one terminus and having been half-n-butyl-esterified. Analysis by infrared absorption spectrometry revealed no residual maleic anhydride ring. As determined by GPC analysis, the $\overline{M}w$ and $\overline{M}n$ values of this copolymer were 1,900 and 1,540, respectively, and the $\overline{M}w$/ $\overline{M}n$ ratio was 1.23.

To 20 ml of 1N aqueous sodium hydroxide solution was added 1.00 g of the above half-n-butyl-esterified styrene-maleic anhydride copolymer and the mixture was stirred at room temperature for 4 hours. This reaction mixture was adjusted to pH 1-2 by gradual addition of 2.5N hydrochloric acid with stirring and the resulting precipitate was recovered by filtration, rinsed and dissolved in 20 ml of acetone. To this solution was added 1 ml of 2.5N hydrochloric acid, whereby a homogenous acidic solution was obtained. This acidic solution was diluted with 30 ml of water and the acetone was distilled off under reduced pressure. The resulting precipitate was recovered by filtration, rinsed and dried. The procedure gave 0.85 g of a styrene-maleic anhydride copolymer having a group of the formula

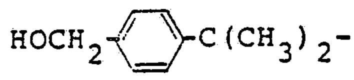

at one terminus and having been half-n-butyl-esterified.
IR absorption spectrum: 3200, 2960, 1740, 1712, 1460, 1170, 708 $cm^{-1}$

(c) Synthesis of a maleimide group-containing copolymer by reaction between 6-(N-maleimido)caproyl chloride and styrene-maleic anhydride copolymer having a group of the formula

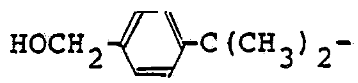

at one terminus and having been half-n-butyl-esterified

To 2.0 ml of tetrahydrofuran were added 283 mg (1.2 m moles) of the 6-(N-maleimido)caproyl chloride obtained in (a) above, 50 μl of pyridine, and 100 mg (0.05 m mole) of the styrene-maleic anhydride copolymer having a group of the formula

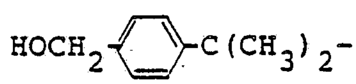

at one terminus and having been half-n-butyl-esterified and the mixture was reacted at room temperature with stirring for 5 hours. The reaction mixture was filtered, and to remove the unreacted 6-(N-maleimido)-caproyl chloride, the filtrate was subjected to preparative GPC [apparatus: LC-9, Japan Analytical Industry Co., Ltd.; columns: JAIGEL IH 20 x 600 mm (dia.) x 2; eluent: tetrahydrofuran]. From the fractions rich in the desired product, the tetrahydrofuran was distilled off under reduced pressure to give 105 mg of a solid reaction product. This product was added to 200 ml of phosphate buffer (pH 8.0) and the mixture was stirred at room temperature for 3 hours, whereby the mixed acid anhydride bond formed by reaction of 6-(N-maleimido)caproyl chloride with the carboxyl group of the material copolymer was hydrolyzed. This reaction mixture was adjusted to pH 1 to 2 with 2.5N hydrochloric acid under stirring. The resulting precipitate was recovered by filtration, rinsed and dissolved in 20 ml of acetone. To this solution was added 0.5 ml of 2.5N hydrochloric acid, whereby a homogeneous acidic solution was obtained. This acidic solution was diluted with 30 ml of water and the acetone was distilled off under reduced pressure. The resulting precipitate was recovered by filtration, rinsed and dried. The procedure gave 80 mg of a styrene-maleic anhydride copolymer having a group of the formula

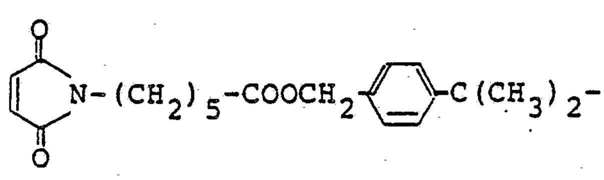 at one terminus

and having been half-n-butyl-esterified. The average maleimide group content per copolymer molecule was 0.4. The infrared absorption spectrum of the above product is shown in Fig. 2.

Example 4

Synthesis of maleimide group-containing copolymer (2)

(a) Synthesis of a styrene-maleic anhydride copolymer having a group of the formula

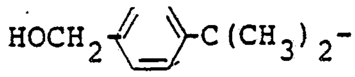

at one terminus and having been half-n-butyl-esterified

A styrene-maleic anhydride copolymer having a group of the formula

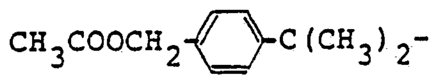

at one terminus as prepared by the procedure set forth in Example 3 (b) was subjected to the above-described fractional precipitation. With the resulting syrupy substance being left in the original vessel, the 2:8 v/v) mixture of acetone and n-hexane was transferred to another vessel, in which the solvents were distilled off under reduced pressure, whereupon 3.0 g of a viscous liquid was obtained. This viscous liquid was dissolved in 45 ml of tetrahydrofuran and the solution was subjected to preparative GPC in the same manner as Example 3 (c). From the eluate containing the desired product, the tetrahydrofuran was distilled off under reduced pressure. The procedure gave 0.90 g of a fractionated styrene-maleic anhydride copolymer having a group of the formula

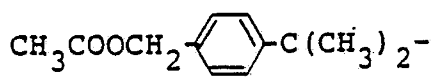

at one terminus. As determined by GPC analysis, the $\overline{M}w$ and $\overline{M}n$ values of this copolymer were 790 and 770, respec tively, and the $\overline{M}w/\overline{M}n$ ratio was 1.03.

The styrene-maleic anhydride copolymer (0.80 g) thus obtained was half-n-butyl-esterified and then hydrolyzed in the same manner as Example 3 (b) to give 0.54 g of a styrene-maleic anhydride copolymer having a group of the formula

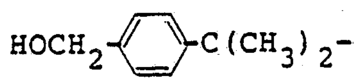

at one terminus and having been half-n-butyl-esterified. As determined by GPC analysis, the $\overline{M}w$ and $\overline{M}n$ values of this product were 1010 and 980, respectively, and the $\overline{M}w/\overline{M}n$ ratio was 1.03.

(b) Synthesis of a maleimide group-containing copolymer by reaction between 6-(N-maleimido)caproyl chloride and a styrene-maleic anhydride copolymer having a group of the formula

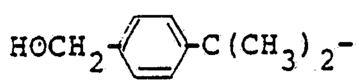

at one terminus and having been half-n-butyl-esterified

Using 0.20 g (0.2 m mole) of a styrene-maleic anhydride copolymer having a group of the formula

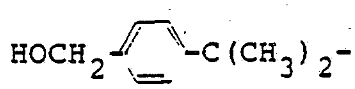

at one terminus and having been half-n-butyl-esterified as obtained by the procedure set forth in (a) above and 0.23 g (1.0 m mole) of 6-(N-maleimido)caproyl chloride as obtained by the procedure of Example 3 (a), the reaction of Example 3 (c) was repeated to give 0.17 g of a styrene-maleic anhydride copolymer having a group of the formula

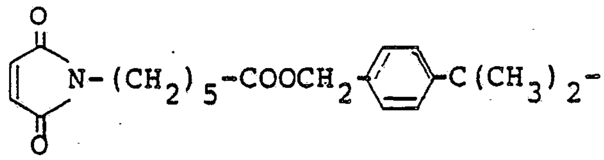

at one terminus and having been half-n-butyl-esterified.

Example 5

Synthesis of maleimide group-containing copolymer (2)

(a) Synthesis of ethylene glycol bis(3-N-maleimidopropyl) ether

To a solution of 21.60 g (0.22 mole) of maleic anhydride in 60 ml of acetone was added a solution of 17.60 g (0.10 mole) of ethylene glycol bis(3-aminopropyl) ether in 90 ml of acetone dropwise over 1 hour at 25-30 °C with constant stirring. After completion of dropwise addition, 0.20 g of lithium bromide, 5 ml of triethylamine and 25.50 g (0.25 mole) of acetic anhydride were added and the reaction mixture was stirred at 60 °C for 2 hours. This reaction mixture was cooled to room temperature and 200 ml of water was added dropwise. The mixture was extracted with methylene chloride and the organic layer was washed with water and dried over anhydrous sodium sulfate. From the organic layer, the solvent was distilled off to give a viscous liquid. To this viscous liquid was added 500 ml of cyclohexane and the mixture was stirred at 50-60 °C for 10 minutes, after which it was allowed to stand for a while. The supernatant was cooled to room temperature to give 0.80 g white crystals of ethylene glycol bis(3-N-maleimidopropyl) ether which had the following physical values.
UV absorption spectrum: $\lambda$max 300 nm
IR absorption spectrum: 3100, 2890, 1700, 1410, 1116, 844, 698 $cm^{-1}$

(b) Synthesis of S-p-isopropylphenyl thioacetate

To 300 ml of chloroform was added 80.0 g (0.67 mole) of isopropylbenzene and the mixture was stirred with cooling on an ice-bath at -10 °C - 0 °C. To this solution, 89 ml (1.34 moles) of chlorosulfonic acid, purified by distillation, was added dropwise over a period of about 30 minutes. After completion of dropwise addition, the mixture was stirred at room temperature for 30 minutes. The reaction mixture was poured in ice-water and extracted with 200 ml of chloroform. The extract was washed 3 times with 500 ml portions of iced water and dried by addition of anhydrous magnesium sulfate. From this extract, the chloroform was distilled off under reduced pressure to give 55.0 g of p-isopropylbenzenesulfonyl chloride (yield based on isopropylbenzene: 38%).

A three-necked flask of 2-liter capacity was charged with 500 g of ice and, then, 100 ml of con centrated sulfuric acid was cautiously added. The resulting aqueous solution was cooled to 0 °C and 55.0 g (0.25 mole) of the p-isopropylbenzenesulfonyl chloride prepared above was added. Then, while the temperature of the solution was maintained at 0-2 °C, zinc dust was added gradually. The reaction was conducted with stirring for 24 hours and, then, on reflux with stirring for an additional 1.5 hours. The reaction mixture was cooled to room temperature and extracted with n-hexane. The extract was dried over anhydrous magnesium sulfate and distilled to give 24.2 g of p-isopropylthiophenol boiling at 106.5-107.5 °C/21 mmHg (yield based on p-isopropylbenzenesulfonyl chloride: 63%).

In 500 ml of diethyl ether was dissolved 24.2 g (0.13 mole) of the above p-isopropylthiophenol, followed

by addition of 10.7 ml (0.14 mole) of acetyl chloride. While the solution was stirred on an ice-bath, 11.4 ml (0.14 mole) of pyridine was added dropwise over 10 minutes. After completion of dropwise addition, the mixture was stirred at room temperature overnight. The resulting reaction mixture was filtered to remove the precipitate. The filtrate was washed with distilled water twice, dried over anhydrous magnesium sulfate and distilled to give 21.6 g of S-p-isopropylphenyl thioacetate boiling at 148-150 °C/20 mmHg (yield based on p-isopropylthiophenol: 85%).

(c) Synthesis of a styrene-maleic anhydride copolymer having a group of the formula

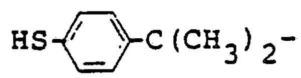

at one terminus and having been half-n-butyl-esterified

In a nitrogen gas stream, 4.30 g (44 m moles) of maleic anhydride was added to 15 ml of the S-p-isopropylphenyl thioacetate obtained in (b) above and the mixture was heated at about 150 °C with stirring. Then, a solution of 0.16 g (0.59 m mole) of dicumyl peroxide and 2.30 g (22 m moles) of styrene in 10 ml of S-p-isopropylphenyl thioacetate was added dropwise to the above solution over a period of about 15 minutes. After addition, the mixture was further stirred under heating for 15 minutes. After cooling to room temperature, 2 ℓ of n-hexane was added dropwise, with stirring, over about 30 minutes. The resulting precipitate was recovered by filtration and dried to give 7.60 g of a styrene-maleic anhydride copolymer having a group of the formula

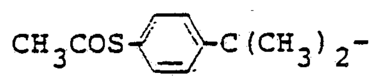

at one terminus.

In 150 ml of acetone was dissolved 7.60 g of the above styrene-maleic anhydride copolymer and, then, 225 ml of n-hexane was added dropwise to the solution with stirring over a period of about 30 minutes. The acetone-n-hexane mixture developing white turbidity was transferred to another vessel and 375 ml of n-hexane was added dropwise to the above mixture with stirring over a period of about 40 minutes. The resulting syrup was dissolved in acetone and recovered. From this recovered solution, the acetone was distilled off under reduced pressure and the residue was dried in vacuo at room temperature overnight. The procedure gave 2.90 g of a fractionally precipitated styrene-maleic anhydride copolymer having a group of the formula

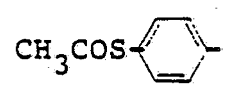

$C(CH_3)_2-$

at one terminus. The $\overline{M}w$ and $\overline{M}n$ values of this product as determined by GPC analysis were 1450 and 1180, respectively. ($\overline{M}w/\overline{M}n \fallingdotseq 1.23$).

To 20 ml of n-butyl alcohol were added 1.00 g of the fractionally precipitated styrene-maleic anhydride copolymer and 10 mg of anhydrous lithium acetate and the mixture was heated at 95 °C with stirring for 20 hours. This reaction mixture was distilled under reduced pressure to remove the n-butyl alcohol. To the residue was added 10 ml of dioxane and the mixture was lyophilized to give 1.20 g of a styrene-maleic anhydride copolymer having a group of the formula

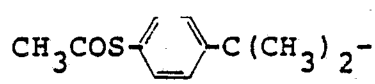

at one terminus and having been half-n-butyl-esterified. Infrared absorption spectrophotometry revealed no residual maleic anhydride ring in this product. The $\overline{M}w$ and $\overline{M}n$ values of the product as determined by GPC analysis were 1950 and 1590, respectively ( $\overline{M}w/\overline{M}n = 1.23$).

To 20 ml of 1N aqueous sodium hydroxide solution was added 0.25 g of the above half-n-butyl-esterified styrene-maleic anhydride copolymer and the mixture was stirred at room temperature for 20 minutes. The reaction mixture was then adjusted to pH 1-2 by gradual addition of 2.5N hydrochloric acid with stirring. The resulting precipitate was recovered by filtration, rinsed and dissolved in 20 ml of acetone. To this solution was added 1 ml of 2.5N hydrochloric acid to give a homogeneous acidic solution. This acidic solution was diluted with 30 ml of water and the acetone was distilled off under reduced pressure. The precipitate was recovered by filtration, rinsed and dried to give 0.23 g of a styrene-maleic anhydride copolymer having a group of the formula

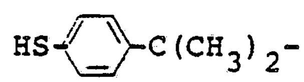

at one terminus and having been half-n-butyl-esterified, the physical data of which are given below.
IR absorption spectrum: 3200, 2960, 1740, 1710, 1455, 1170, 705 $cm^{-1}$

(d) Synthesis of a maleimide-containing copolymer by reaction between ethylene glycol bis(3-N-maleimidopropyl)ether and a styrene-maleic anhydride copolymer having a group of the formula

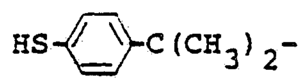

at one terminus and having been half-n-butyl-esterified

In 1 ml of acetonitrile was dissolved 40 mg (1.2 x $10^{-4}$ moles) of the ethylene glycol bis(3-N-maleimidopropyl) ether obtained in (a) above and while the solution was stirred at room temperature, 40 mg (2 x $10^{-5}$ moles) of the styrene-maleic anhydride copolymer having a group of the formula

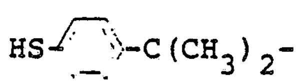

at one terminus and having been half-n-butyl-esterified as obtained in (c) above was added gradually over a period of about 5 minutes. After completion of the addition, the mixture was further stirred for 5 hours. The reaction mixture was filtered and, for removal of unreacted ethylene glycol bis(3-N-maleimidopropyl) ether, the filtrate was subjected to preparative GPC (apparatus: LC-9, Japan Analytical Industries Co., Ltd.; column: JAIGEL 1H, 20 x 600 mm dia. x 2; eluent: chloroform). From the eluate, the chloroform was distilled off under reduced pressure to give 28 mg of a styrene-maleic anhydride copolymer having a group of the formula

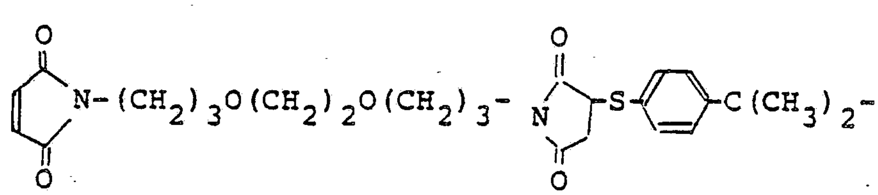

at one terminus and having been half-n-butyl-esterified. The average maleimide group content of this copolymer was 0.6 per copolymer molecule. The infrared absorption spectrum of this product is shown in Fig. 3.

Example 6

Synthesis of maleimide group-containing copolymer (2)

(a) Synthesis of N,N′-hexamethylenebismaleimide

To a solution of 11.60 g (0.10 mole) of 1,6-diaminohexane in 50 ml of chloroform was added a solution of 19.60 g (0.20 mole) of maleic anhydride in 100 ml of chloroform dropwise over a period of about 1 hour. After completion of dropwise addition, the reaction was conducted under reflux of chloroform for 4 hours. The resulting reaction mixture was distilled under reduced pressure to remove the chloroform, and 7.5 g of anhydrous sodium acetate and 100 ml of acetic anhydride were added to the residue. The mixture was stirred at 90° C for 4 hours, after which it was cooled to room temperature. The resulting precipitate was filtered off and the solvent was distilled off under reduced pressure to give a viscous liquid. This liquid was subjected to silica gel column chromatography using a 2:8 (v/v) mixture of acetone and n-hexane as the eluent, whereby crude crystals were obtained. The crude crystals were recrystallized from benzene to give 1.37 g of N,N′-hexamethylenebismaleimide, physical constants of which are shown below.
Melting point: 136-137° C
UV absorption spectrum: $\lambda_{max}$ 300 nm
IR absorption spectrum: 3000, 2950, 1700, 1450, 1410, 840, 700 $cm^{-1}$

(b) Synthesis of a maleimide group-containing copolymer by reaction between N,N′-hexamethylenebismaleimide and a styrene-maleic anhydride copolymer having a group of the formula

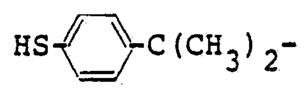

at one terminus and having been half-n-butyl-esterified

In 10 ml of acetonitrile was dissolved 199 mg (7.2 x $10^{-4}$ moles) of the N,N′-hexamethylenebismaleimide obtained in (a) above. To this solution was added 240 mg (1.2 x $10^{-4}$ moles) of a styrene-maleic anhydride copolymer having a group of the formula

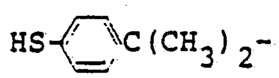

at one terminus and having been half-n-butyl-esterified, prepared in the same manner as Example 5-(c), dropwise over a period of about 5 minutes. After completion of dropwise addition, the mixture was stirred for a further 5 hours. The reaction mixture was then distilled under reduced pressure to remove the acetonitrile and the residue was diluted with 3 ml of chloroform and filtered. For removal of unreacted N,N′-hexamethylenebismaleimide, the filtrate was subjected to preparative GPC in the same manner as Example 5-(d). The procedure gave 120 mg of a styrene- maleic anhydride copolymer having a group of the formula

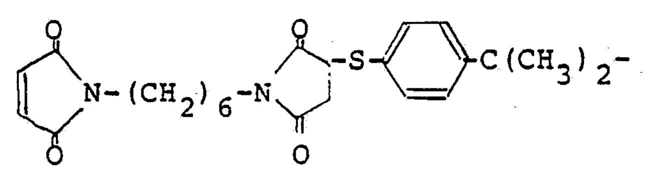

at one terminus and having been half-n-butyl-esterified. The average maleimide group content of this copolymer was 0.5 per copolymer molecule. The infrared absorption spectrum of this copolymer is shown

in Fig. 4.

## Example 7

Synthesis of a maleimide group-containing copolymer (1)

The procedure of Example 1-(b) was repeated except that 3.02 g of n-hexyl alcohol was used in lieu of 2.20 g of n-butyl alcohol to obtain 9.8 g of a styrene-maleic anhydride copolymer having been partially n-hexyl-esterified. Then, in the same manner as Example 1-(c), 116 mg of N-(6-aminohexyl)maleimide hydrochloride was reacted with 850 mg of the styrene-maleic anhydride copolymer having been partially half-n-hexyl-esterified to give 550 mg of the desired maleimide group-containing copolymer. This product was a mixture of a copolymer having a group of the formula

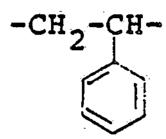

as constituent unit (a), a group of the formula

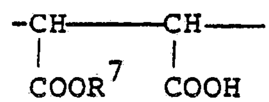

(wherein $R^7$ is n-hexyl or hydrogen) as constituent unit (b) and a group of the formula

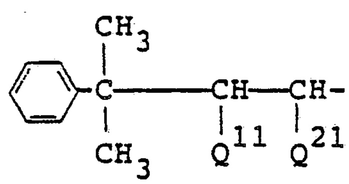

(wherein one of $Q^{11}$ and $Q^{21}$ is carboxy, with the other being a group of the formula

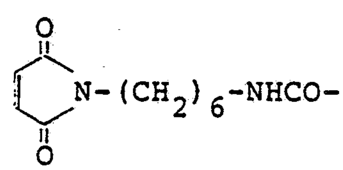

at one terminus and a copolymer having a group of the formula

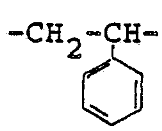

as constituent unit (a), a group of the formula

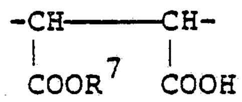

(wherein $R^7$ is n-hexyl or hydrogen) as constituent unit (b) and a group of the formula

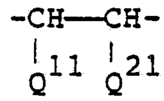

(wherein one of $Q^{11}$ and $Q^{21}$ is carboxy, with the other being a group of the formula

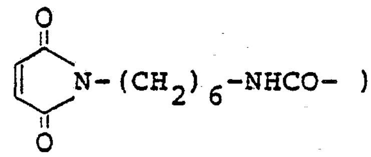

between said constituent units. The average maleimide group content of this product was 0.5 per copolymer molecule.

## Example 8

Synthesis of a maleimide group-containing copolymer (3)

To 20 ml of ethylbenzene was added 2.16 g of maleic anhydride and the mixture was stirred at about 110 °C. To this solution was added, dropwise over about 15 minutes, a solution prepared by dissolving 108 mg of azoisobutyronitrile, 2.08 g of styrene and 289 mg of S-4-mercaptobutyl thioacetate in 10 ml of ethylbenzene. After completion of dropwise addition, the mixture was further stirred for 15 minutes and, then, cooled. This reaction mixture was added dropwise to 2 ℓ of hexane over about 30 minutes, with constant stirring. The resulting precipitate was recovered by filtration, washed with hexane and dried to give 4.10 g of a styrene-maleic anhydride copolymer having a group of the formula $CH_3COS-(CH_2)_4-S-$ at one terminus.

To 40 ml of isopentyl alcohol were added 2.00 g of the above copolymer and 20 mg of anhydrous lithium acetate and the mixture was stirred at 95 °C for 20 hours. From the reaction mixture thus obtained, the isopentyl alcohol was distilled off under reduced pressure. The residue was diluted with 20 ml of dioxane and lyophilized to give 2.38 g of a styrene-maleic anhydride copolymer having a group of the formula $CH_3COS-(CH_2)_4-S-$ at one terminus and having been half-isopentyl-esterified.

In the same manner as described in Example 5 (c) for the treatment of a styrene-maleic anhydride copolymer having a group of the formula

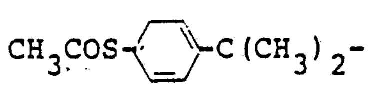

at one terminus and having been half-n-butyl-esterified with an 1N-aqueous sodium hydroxide solution, 0.25 g of the styrene-maleic anhydride copolymer having a group of the formula $CH_3COS-(CH_2)_4-S-$ at one terminus and having been half-isopentyl-esterified was treated with 20 ml of 1N-aqueous sodium hydroxide solution to give 0.21 g of a styrene-maleic anhydride copolymer having a group of the formula $HS-(CH_2)_4-S-$ at one terminus and having been half-isopentyl-esterified.

Then, in the same manner as Example 5 (d), 40 mg of the styrene-maleic anhydride copolymer having a group of the formula $HS-(CH_2)_4-S-$ at one terminus and having been half-isopentyl-esterified was reacted

with 40 mg of ethylene glycol bis(3-N-maleimidopropyl) ether to give 24 mg of a styrene-maleic anhydride copolymer having a group of the formula

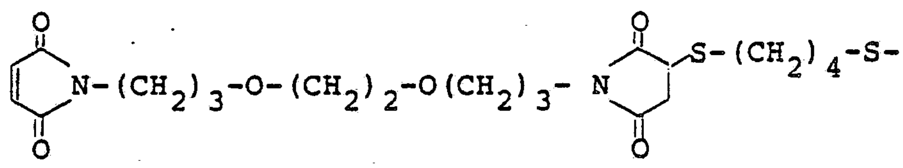

at one terminus and having been half-isopentyl-esterified. The average maleimide group content of this product was 0.4 per copolymer molecule.

## Example 9

Synthesis of an SOD derivative by reaction between SOD and maleimide group-containing copolymer (1) of Example 1

In 2.5 ml of 0.1N-aqueous Tris-hydrochloric acid solution (pH 8.0) was dissolved 360 µl of an aqueous solution of human SOD (87 mg/ml). To this solution was added, gradually under stirring, a solution of 36.6 mg of the maleimide group-containing copolymer of Example 1 in 150 µl of dimethyl sulfoxide. The mixture was stirred at room temperature for 3 hours and, then, allowed to stand at 4°C overnight. The reaction mixture was filtered and the filtrate was subjected to gel filtration on Sephadex G-75 (Pharmacia Fine Chemicals) using 20 mM phosphate buffer (pH 7.2) as the eluent. The SOD-rich and SOD derivative-rich fractions were pooled and subjected to ultrafiltration to give 3 ml of a concentrate. The protein content of this concentrate was 7.7 mg/ml. A 500 µl portion of the concentrate was subjected to affinity column chromatography on AH-Sepharose 4B (Pharmacia Fine Chemicals) (gel: 2 ml) and elution was carried out with 10 ml of 10 mM phosphate buffer containing 0.1 M sodium hydrogen carbonate to obtain a fraction containing unreacted SOD. Then, the column was further eluted with 10 ml of 10 mM phosphate buffer containing 0.1 M sodium hydrogen carbonate and 3 M sodium chloride to obtain a fraction containing the SOD derivative. This SOD derivative-containing fraction was concentrated to 200 µl by ultrafiltration. The yield of the SOD derivative was 1 mg. Fig. 5 (a), (b), (c), (d) and (e) show the electrophoretograms of the starting material SOD, the reaction mixture obtained as above, the fraction containing SOD and SOD derivative as obtained by gel filtration, the SOD fraction obtained by affinity column chromatography, and the SOD derivative fraction obtained by affinity column chromatography, respectively. Fig. 6 (1), (2) and (3) show the ultraviolet absorption spectra of the starting material SOD, the starting material maleimide-containing copolymer and the product SOD derivative, respectively.

## Example 10

Synthesis of an SOD derivative by reaction between SOD and maleimide group-containing copolymer (2) of Example 3

To 2.5 ml of phosphate buffer (pH 7.5) was added 150 µl ($6.4 \times 10^{-7}$ mole) of an aqueous solution of human SOD (68 mg/ml) and the mixture was stirred at 5-10°C. To this solution was gradually added 25.0 mg ($1.2 \times 10^{-5}$ moles) of the maleimide group-containing copolymer (2) of Example 3 and the mixture was further stirred for 20 hours. The reaction mixture was filtered and the filtrate was subjected to gel permeation chromatography on Sephadex G-75SF (Pharmacia Fine Chemicals) (column: K26/40, Pharmacia Fine Chemicals) using 10 mM aqueous ammonium bicarbonate as the eluent. The SOD derivative fraction was lyophilized to recover 11.2 mg of the desired SOD derivative. The infrared absorption spectrum of this product is shown in Fig. 7.

Example 11

Synthesis of an SOD derivative by reaction between SOD and the maleimide group-containing copolymer (2) of Example 3

To 400 µl of an aqueous solution of human SOD (87 mg/ml) was added 400 µl of 0.25 M aqueous potassium monohydrogen phosphate to establish a pH of 8.0. To this aqueous solution was added, gradually under stirring, a solution prepared by dissolving 43.0 mg of the maleimide group-containing copolymer (2) of Example 3 in 100 µl of dimethyl sulfoxide. After completion of the addition, the reaction mixture was adjusted to pH 8.0 with 1 M aqueous Tris solution and the mixture was stirred at room temperature for 3 hours and, then, allowed to stand at 4° C overnight. The reaction mixture was filtered and the filtrate was subjected to gel permeation chromatography on Sephadex G-50 (Pharmacia Fine Chemicals) using 20 mM phosphate buffer (pH 7.2) as the eluent. The fractions containing SOD and SOD derivatives were pooled and concentrated to about 1.3 ml by ultrafiltration. The protein content of the concentrate was 23.6 mg/ml. A 1.2 ml portion of this concentrate was subjected to affinity column chromatography on AH-Sepharose 4B (Pharmacia Fine Chemicals) gel: 15 ml) and elution was carried out with 45 ml of 0.1 M Tris-HCl solution as the eluent to recover a fraction containing unreacted SOD. Then, elution was further carried out with 45 ml of 0.1 M Tris-HCl solution (pH 8.0) containing 1 M sodium chloride to recover a fraction containing the SOD derivative. This SOD derivative fraction was subjected to ultrafiltration and the concentrate was dialyzed against 20 mM phosphate buffer (pH 7.2) (1.5 ℓ x 3 times), whereby 1.6 ml of a solution containing the SOD derivative was obtained. The yield of the SOD derivative was 17 mg. Fig. 8 (a), (b), (c), (d) and (e) show the electrophoretograms of the starting material SOD, the reaction mixture obtained as above, the pooled fraction containing SOD and SOD derivative as obtained by gel permeation chromatography, the SOD fraction obtained by affinity column chromato graphy and the SOD derivative fraction obtained by affinity column chromatography, respectively.

Example 12

Synthesis of an SOD derivative by reaction between SOD and maleimide group-containing copolymer (2) of Example 4

The reaction and isolation procedures of Example 10 were repeated except that 13.0 mg ($1.3 \times 10^{-5}$ moles) of the maleimide group-containing copolymer (2) of Example 4 was used in lieu of 25.0 mg of the maleimide group-containing copolymer of Example 3. In this manner, 8.8 mg of an SOD derivative was obtained.

Example 13

Synthesis of an SOD derivative by reaction between SOD and maleimide group-containing copolymer (2) of Example 5

The reaction and isolation procedures of Example 10 were repeated except that 29.2 mg ($1.3 \times 10^{-5}$ moles) of the maleimide group-containing copolymer (2) of Example 5 was used in lieu of 25.0 mg of the maleimide group-containing copolymer of Example 3. In this manner, 10.3 mg of an SOD derivative was obtained.

Example 14

Synthesis of an SOD derivative by reaction between SOD and maleimide group-containing copolymer (2) of Example 6

The reaction and purification procedures of Example 10 were repeated except that 26.0 mg ($1.3 \times 10^{-5}$ moles) of the maleimide group-containing copolymer (2) of Example 6 was used in lieu of 25.0 mg of the maleimide group-containing copolymer (2) of Example 3 to obtain 9.8 mg of an SOD derivative. Fig. 9 (1), (2) and (3) show the ultraviolet absorption spectra of the starting material SOD, the starting material maleimide group-containing copolymer, and the product SOD derivative, respectively. The infrared absorption spectrum of the product SOD derivative is shown in Fig. 10.

Example 15

Synthesis of an SOD derivative by reaction between SOD and maleimide group-containing copolymer (1) of Example 7

The reaction and isolation procedures of Example 9 were repeated except that 45.0 mg of the maleimide group-containing copolymer (1) of Example 7 was used in lieu of 36.6 mg of the maleimide group-containing copolymer (1) of Example 1 to give 35.8 mg of an SOD derivative.

Example 16

Synthesis of an SOD derivative by reaction between SOD and maleimide group-containing copolymer (3) of Example 8

The reaction and isolation procedures of Example 10 were repeated except that 30 mg of the maleimide group-containing copolymer (3) of Example 8 was used in lieu of 25.0 mg of the maleimide group-containing copolymer (2) of Example 3 to give 13.8 mg of an SOD derivative.

Test Example 1

Blood concentration of the SOD derivative

After SOD and the SOD derivative obtained in Example 9 were respectively dissolved in distilled water, 25 $\mu$Ci of $^{125}$I-Bolton-Hunter reagent was added to each solution and the reaction was conducted at room temperature under shaking for 1 hour. The reaction mixture thus obtained was allowed to stand at 4°C overnight and, then, dialyzed against 20 mM phosphate buffer (pH 7.3) containing 0.15 M sodium chloride (5 $\ell$ x 3 times). The dialysate was measured for radioactivity per unit volume and, then, diluted with physiological saline solution to a predetermined concentration. A cannula was inserted into the femoral vein of each rat (SD strain, male, 7 weeks of age, body.weight about 200 g) under pentobarbital anesthesia and 0.2 ml of heparin solution (1,000 U/ml) was infused. Then, 200,000 cpm (500 $\mu$l)/rat of the above solution of labeled SOD or labeled SOD derivative was injected into the femoral vein. Thereafter, the blood was withdrawn in 100 $\mu$l portions from the femoral vein at timed intervals and the blood concentration was measured by counting the radioactivity with a gamma counter. The time courses of the blood concentrations of SOD and SOD derivative are shown in Fig. 11 (1) and (2), respectively.

Test Example 2

Effect of the SOD derivative on rat coronary artery reperfusion arrhythmia

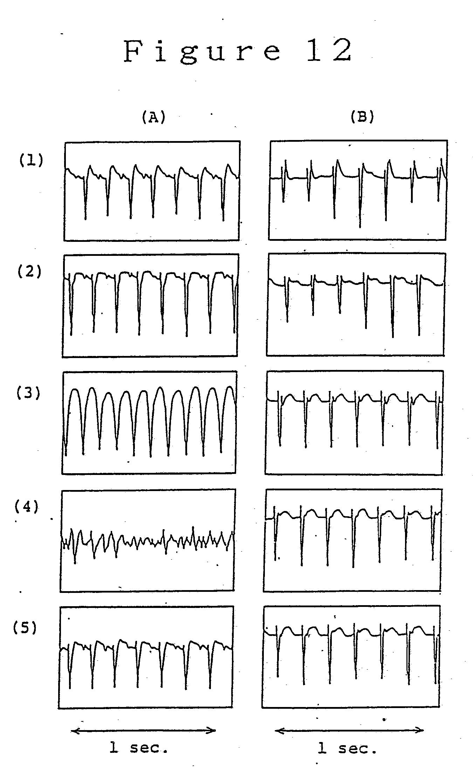

Rats (Wistar strain, male, body weights 200-230 g) were fasted overnight and, then, anesthetized with intraperitoneal pentobarbital (50 mg/kg body weight). After tracheotomy, a respirator was installed and under respiratory control with the respirator (ventilation volume/time = 1.5 $cm^3$/100 g body weight; 60 times/min), the heart was exposed by median sternotomy. The coronary artery was occluded for 15 minutes by aspiration of the anterior descending branch of the left coronary artery at a site about 3 mm peripheral of the origin of the ramus circumflexus. The ischemia-reperfusion procedure was performed twice at a 30-minute interval, and 15 minutes after the first reperfusion, each drug was administered into the femoral vein in a dose of 5 mg/kg body weight (total volume 0.1 ml). As the drugs, SOD isolated from human erythrocytes and the SOD derivative obtained in Example 9 were used as dissolved in physiological saline. In each reperfusion procedure, the electrocardiogram (lead II) was continuously monitored for 3 minutes and the premature ventricular contraction (PVC), ventricular tachycardia (VT) and ventricular fibrillation (Vf) were investigated. The electrocardiograms(ECGS) recorded before the second ischemia, 15 minutes after the second ischemia, and 10 and 30 seconds and 3 minutes after the second reperfusion are shown in the horizontal rows (1), (2), (3), (4) and (5), respectively, of Fig. 12. In Fig. 12, the ECGS in the group given the above human SOD are shown in the vertical row (A) and those in the group given the SOD derivative of Example 9 are shown in the vertical row (B).

In the drug-free control, there was no significant difference in the onset of arrhythmias between the two reperfusions. It is apparent from Fig. 12 that whereas the SOD-treated group showed no inhibition of reperfusion arrhythmia, the group treated with the SOD derivative showed a marked antiarrythmic effect of the derivative.

## Preparation Example 1

Enteric capsules were prepared using the following ingredients in accordance with the established pharma ceutical procedure.

| | |
|---|---|
| The SOD derivative of Example 9 | 20 mg |
| Lactose | 46 mg |
| Corn starch | 16 mg |
| Crystalline cellulose | 12 mg |
| Cellulose glycolate | 5 mg |
| Sorbitol | 10 mg |
| Total (per capsule) | 109 mg |

To administer the above enteric capsules to humans, usually 2 capsules per adult are administered 3 times a day after meals.

## Preparation Example 2

Enteric tablets were prepared using the following ingredients in accordance with the established pharmaceutical procedure.

| | |
|---|---|
| The SOD derivative of Example 9 | 20 mg |
| Lactose | 79 mg |
| Corn starch | 45.5 mg |
| Magnesium stearate | 0.5 mg |
| Total (per tablet) | 145 mg |

To administer the above enteric tablets to humans, usually 2 tablets per adult are administered three

times a day after meals.

Preparation Example 3

A parenteral preparation was prepared in the following manner.

In physiological saline was dissolved 10 mg of the SOD derivative obtained in Example 9 to make 5 ml and the solution was filtered through a sterile micropore filter. The filtrate was sealed into a sterile amber-colored vial (Type I, 3 ml).

## Claims

1. A copolymer which comprises the polymer-constituting units (a) and (b): wherein (a) is a group of the formula

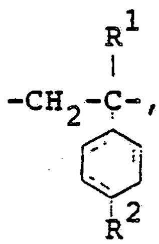, 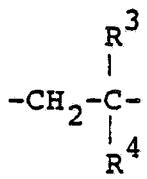, 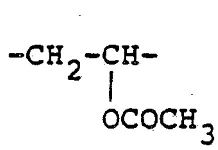 or 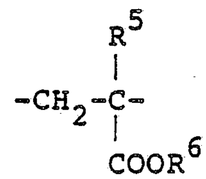

wherein $R^1$, $R^3$ and $R^5$ each independently is a hydrogen atom or a methyl group, $R^2$ is a hydrogen, chlorine or bromine atom or a methyl group, $R^4$ is a hydrogen atom, an alkyl group of 1 to 6 carbon atoms or a cycloalkyl group of 3 to 6 carbon atoms and $R^6$ is a methyl or ethyl group; and wherein (b) is a group of the formula

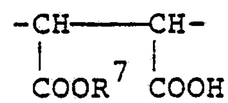

wherein $R^7$ is a hydrogen atom or the residue of an alkanol of 1 to 8 carbon atoms, of an ethylene glycol monoalkyl ether whose alkyl moiety contains 1 to 4 carbons atoms or of a glycerol dialkyl ether whose alkyl moieties each contains 1 to 4 carbon atoms as derived by removal of the hydroxyl group therefrom; and which further comprises:
(c) (i) internally, a group of the formula

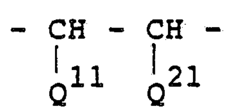

wherein one of $Q^{11}$ and $Q^{21}$ is a carboxyl group and the other is a group of the formula

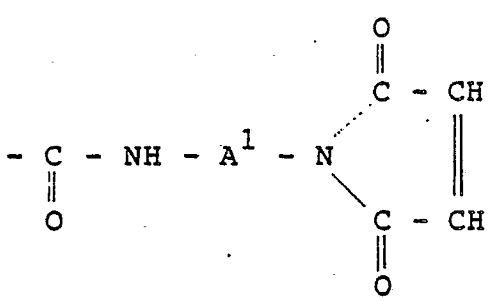

in which $A^1$ is a bivalent hydrocarbon residue which may optionally be interrupted by one or more groups each independently being
an oxygen atom, a sulfur atom, a group of the formula $-N(R^8)-$ ($R^8$ being a hydrogen atom or an alkyl group of 1 to 4 carbon atoms) or a group of the formula -NH-CO-NH-; or
(ii) at one terminus, a group of the formula

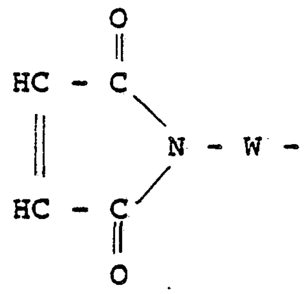

wherein W is a bivalent organic group;
said copolymer having an average molecular weight of 400 to 20,000.

2. The copolymer of Claim 1, wherein the group of the formula

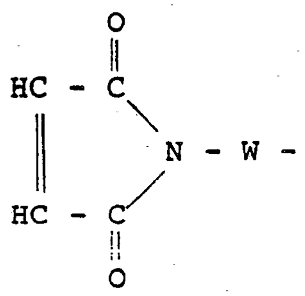

is a group of the formula

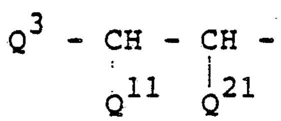

in which $Q^{11}$ and $Q^{21}$ are as defined in Claim 1 and $Q^3$ is a univalent organic group.

3. The copolymer of Claim 1, wherein W is a group of the formula

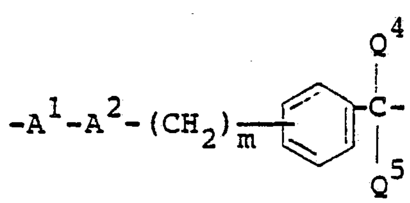

in which $A^1$ is as defined in Claim 1, $A^2$ is -CO-O-, -CO-NH-, $-CO-NH-CH_2-$, -NH-CO-O-, -NH-CO-NH-, $-NH-CO-NH-CH_2-$, -N=CH-, $-NH-CH_2$ or

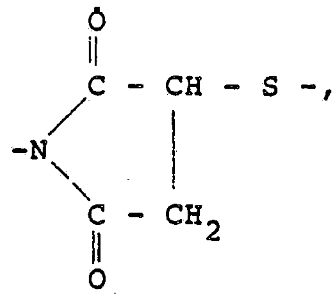

$Q^4$ and $Q^5$ each independently is a hydrogen atom or an alkyl group of 1 to 3 carbon atoms and m is an integer of 0 to 4, the group $A^1$ being bound to the nitrogen atom of the maleimido group, or W is a group of the formula

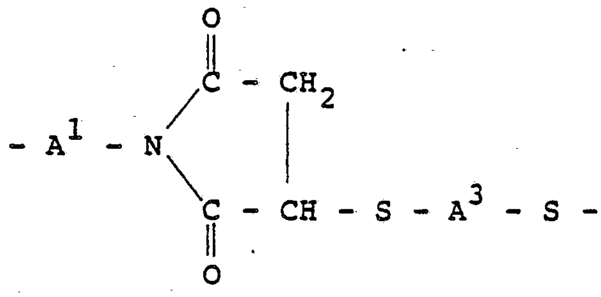

wherein $A^1$ is as defined above and $A^3$ is a bivalent hydrocarbon group which may optionally be interrupted by one or more oxygen and/or sulfur atoms, the group $A^1$ being bound to the nitrogen atom of the maleimido group.

4. A superoxide dismutase derivative of the formula

$[SOD][Z]_n$

wherein [SOD] is a univalent or divalent superoxide dismutase residue derived by hydrogen atom removal from one or two mercapto groups thereof, n is an integer of 1 or 2 corresponding to the valence of [SOD] and [Z] is a univalent group derived from a copolymer according to any one of claims 1 to 3.

5. A method of producing superoxide dismutase derivatives of the formula

$[SOD][Z]_n$

wherein [SOD] is a univalent or divalent superoxide dismutase residue derived by hydrogen atom removal from one or two mercapto groups thereof, n is an integer of 1 or 2 corresponding to the valence of [SOD] and [Z] is a univalent group derived from a copolymer according to any one of claims 1 to 3 which comprises reacting in an aqueous solution having a pH of 6 to 10 the superoxide dismutase with said copolymer.

6. A pharmaceutical composition for the treatment of inflammation and/or ischemic disease which comprises an effective amount for the treatment of said inflammation and/or ischemic disease of the superoxide dismutase derivative of Claim 4 and a pharmacologically acceptable diluent or carrier.

7 . A medicament which contains the superoxide dismutase derivative of Claim 4.

8. A method of producing copolymers having an average molecular weight of 400 to 20,000 and comprising the polymer-constituting units (a) and (b):
wherein (a) is a group of the formula

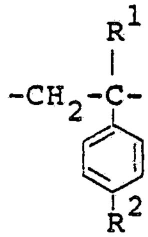, 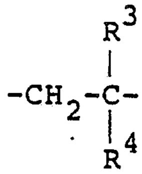, 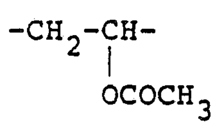 or 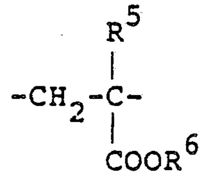

wherein $R^1$, $R^3$ and $R^5$ each independently is a hydrogen atom or a methyl group, $R^2$ is a hydrogen,

chlorine or bromine atom or a methyl group, $R^4$ is a hydrogen atom, an alkyl group of 1 to 6 carbon atoms or a cycloalkyl group of 3 to 6 carbon atoms and $R^6$ is a methyl or ethyl group; and
wherein (b) is a group of the formula

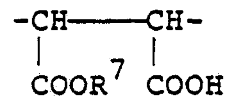

wherein $R^7$ is a hydrogen atom or the residue of an alkanol of 1 to 8 carbon atoms, of an ethylene glycol monoalkyl ether whose alkyl moiety contains 1 to 4 carbons atoms or of a glycerol dialkyl ether whose alkyl moieties each contains 1 to 4 carbon atoms as derived by removal of the hydroxyl group therefrom; and which further comprises:
(c) at one terminus which is a group of the formula

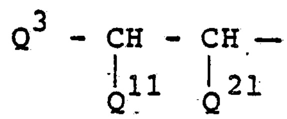

wherein one of $Q^{11}$ and $Q^{21}$ is a carboxyl group and the other is a group of the formula

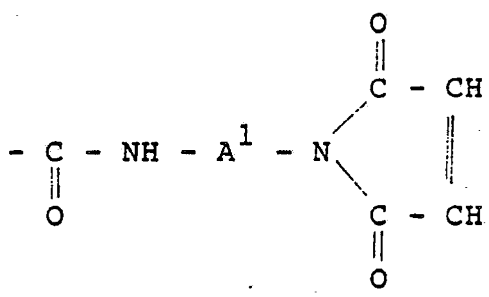

in which $A^1$ is a bivalent hydrocarbon residue which may optionally be interrupted by one or more groups each independently being
an oxygen atom, a sulfur atom, a group of the formula $-N(R^8)-$ ($R^8$ being a hydrogen atom or an alkyl group of 1 to 4 carbon atoms) or a group of the formula -NH-CO-NH-, and $Q^3$ is a univalent organic group; or an internal group of the formula

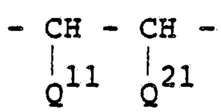

wherein $Q^{11}$ and $Q^{21}$ are as defined above, which method comprises reacting a compound of the formula

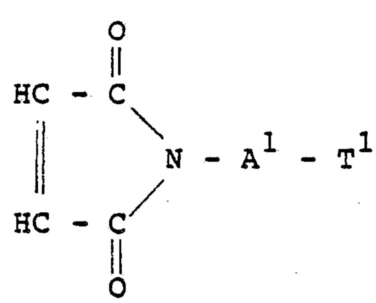

wherein $A^1$ is as defined above and $T^1$ is a group reactive with the maleic anhydride ring, with a copolymer having an average molecular weight of 400 to 20,000 and comprising the polymer constituent units (a) and (b):

wherein (a) is a group of the formula

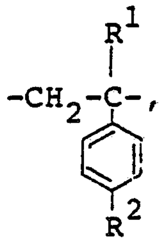, 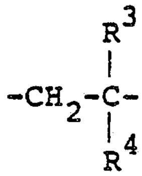, 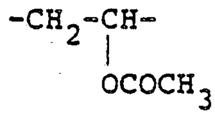 and 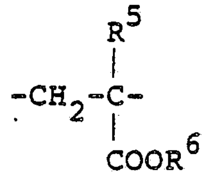

wherein $R^1$, $R^2$, $R^3$, $R^4$, $R^5$ and $R^6$ are as defined above; and
wherein (b) is a group of the formula

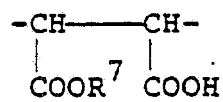

wherein $R^7$ is as defined above; and further comprising:
(c) 0.5 to 2 groups (per molecule) of the formula

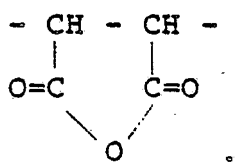.

9. A method of producing copolymers having an average molecular weight of 400 to 20,000 and comprising the polymer-constituting units (a) and (b):
wherein (a) is a group of the formula

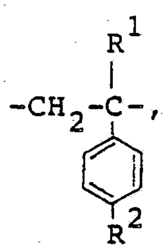, 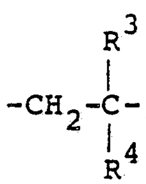, 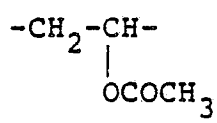 or 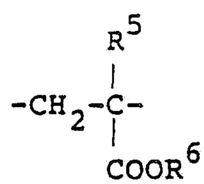

wherein $R^1$, $R^3$ and $R^5$ each independently is a hydrogen atom or a methyl group, $R^2$ is a hydrogen, chlorine or bromine atom or a methyl group, $R^4$ is a hydrogen atom, an alkyl group of 1 to 6 carbon atoms or a cycloalkyl group of 3 to 6 carbon atoms and $R^6$ is a methyl or ethyl group; and
wherein (b) is a group of the formula

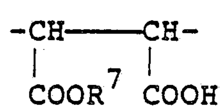

wherein $R^7$ is a hydrogen atom or the residue of an alkanol of 1 to 8 carbon atoms, of an ethylene glycol monoalkyl ether whose alkyl moiety contains 1 to 4 carbons atoms or of a glycerol dialkyl ether whose alkyl moieties each contains 1 to 4 carbon atoms as derived by removal of the hydroxyl group therefrom; and further comprising:
at one terminus, a group of the formula

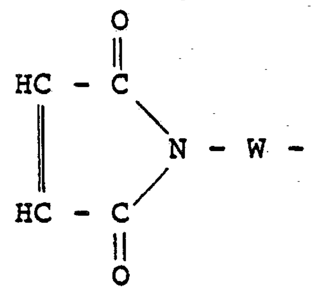

wherein W is a bivalent organic group, which method comprises half-esterifying with an alkanol of 1 to 8 carbon atoms, an ethylene glycol monoalkyl ether whose alkyl moiety contains 1 to 4 carbon atoms or a glycerol dialkyl ether whose alkyl moieties each independently contains 1 to 4 carbon atoms and/or hydrolyzing a copolymer obtained by radical-copolymerizing

(a′) a monomer selected from the class consisting of compounds of the formulas

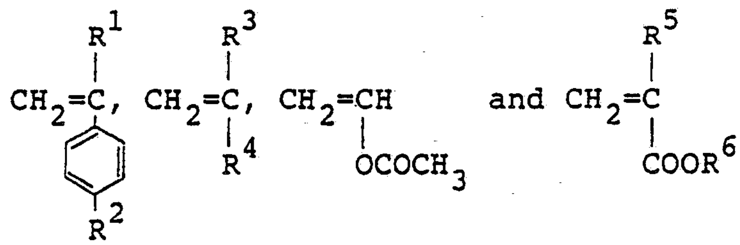

wherein $R^1$, $R^2$, $R^3$, $R^4$, $R^5$ and $R^6$ are as defined above and

(b′) maleic anhydride in the presence of a polymerization initiator having a functional group or a protected functional group and/or a chain transfer agent having a functional group or a protected functional group, and having, at one terminus, said functional group or protected functional group and an average molecular weight of 400 to 20,000; then eliminating the protective group from the terminal protected functional group of said copolymer if necessary, and reacting the thus-modified copolymer with a compound of the formula

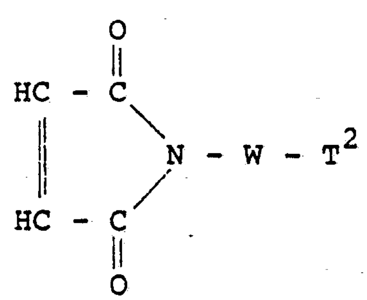

wherein W is a bivalent organic group and $T^2$ is a group reactive with the functional group of said modified copolymer.

Figure 1

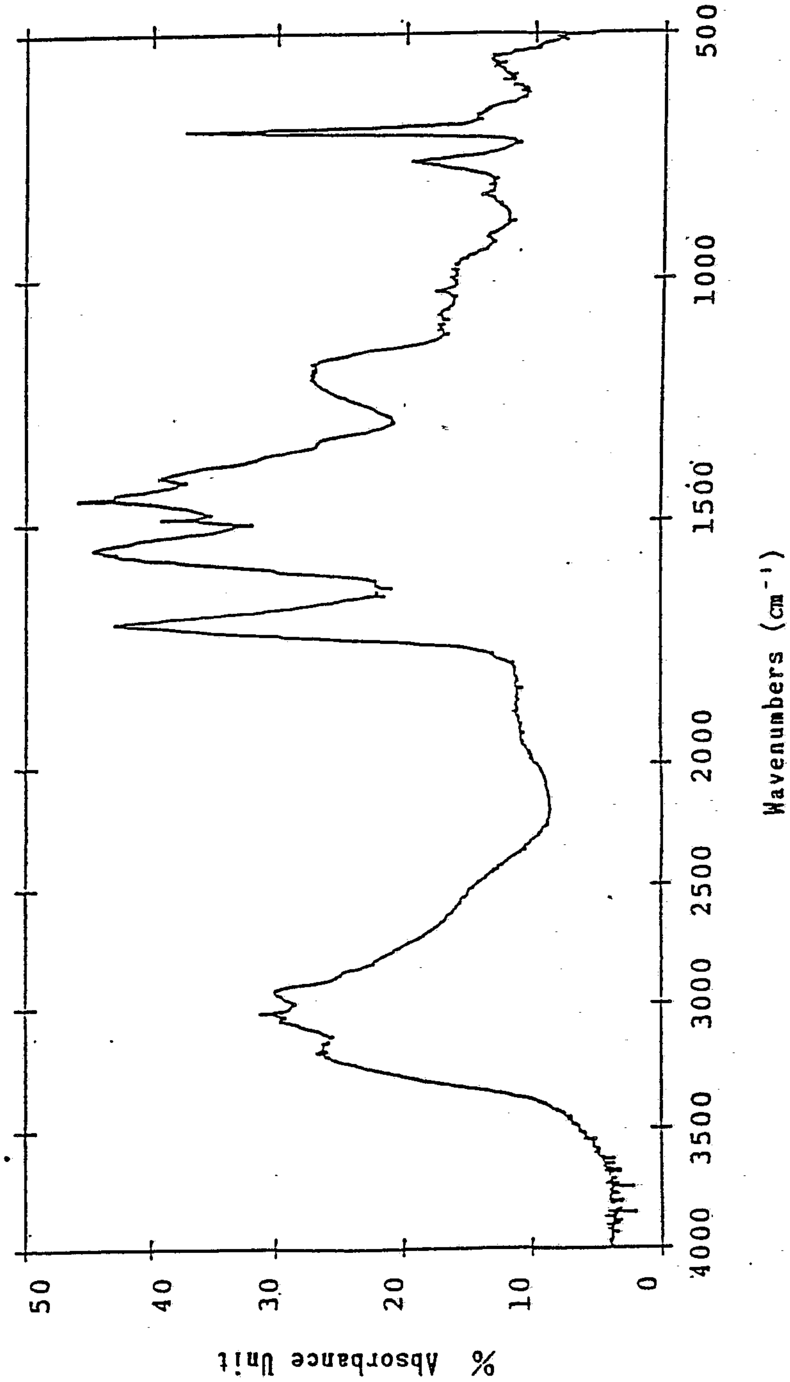
% Absorbance Unit
50
40
30
20
10
0
4000
3500
3000
2500
2000
1500
1000
500
Wavenumbers (cm⁻¹)

Figure 2

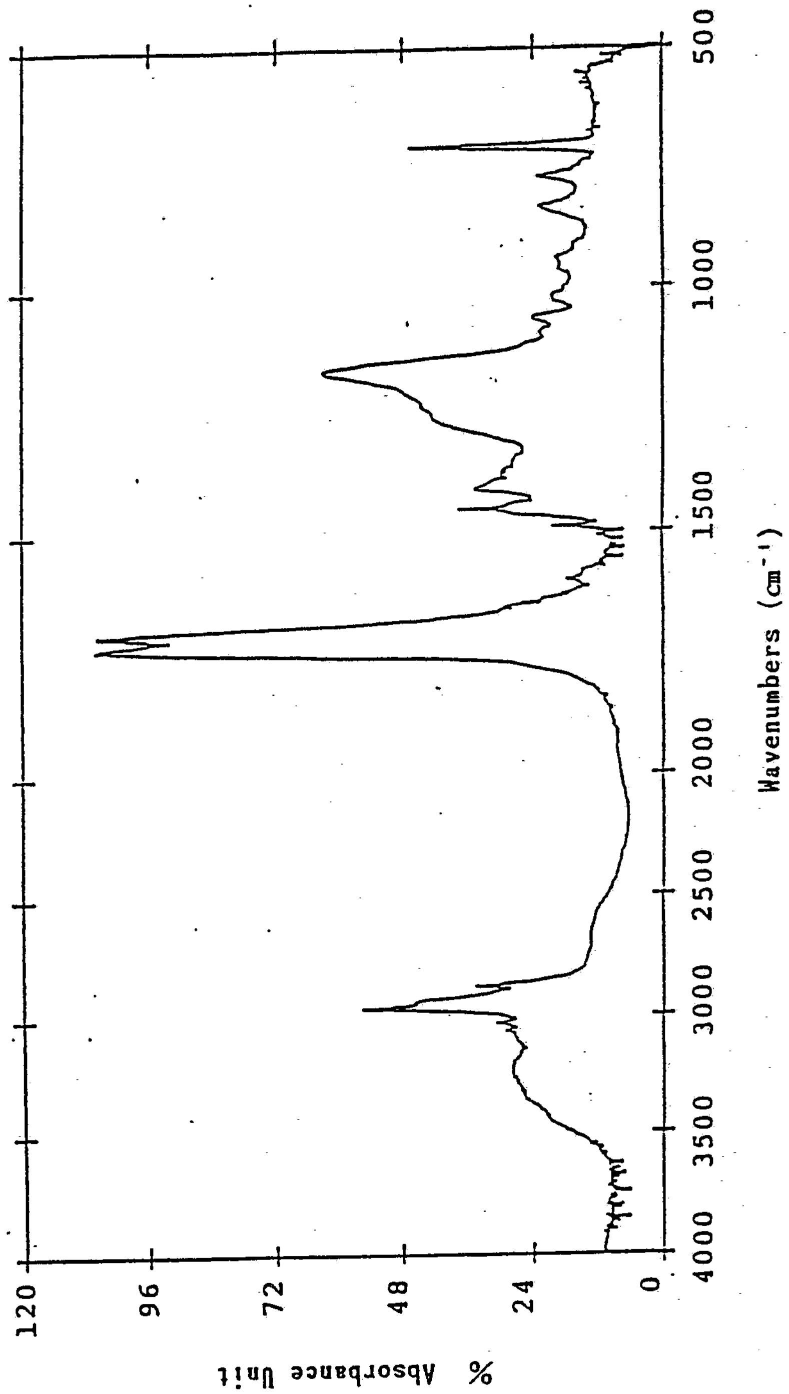
% Absorbance Unit
120
96
72
48
24
0
4000
3500
3000
2500
2000
1500
1000
500
Wavenumbers ($cm^{-1}$)

Figure 3

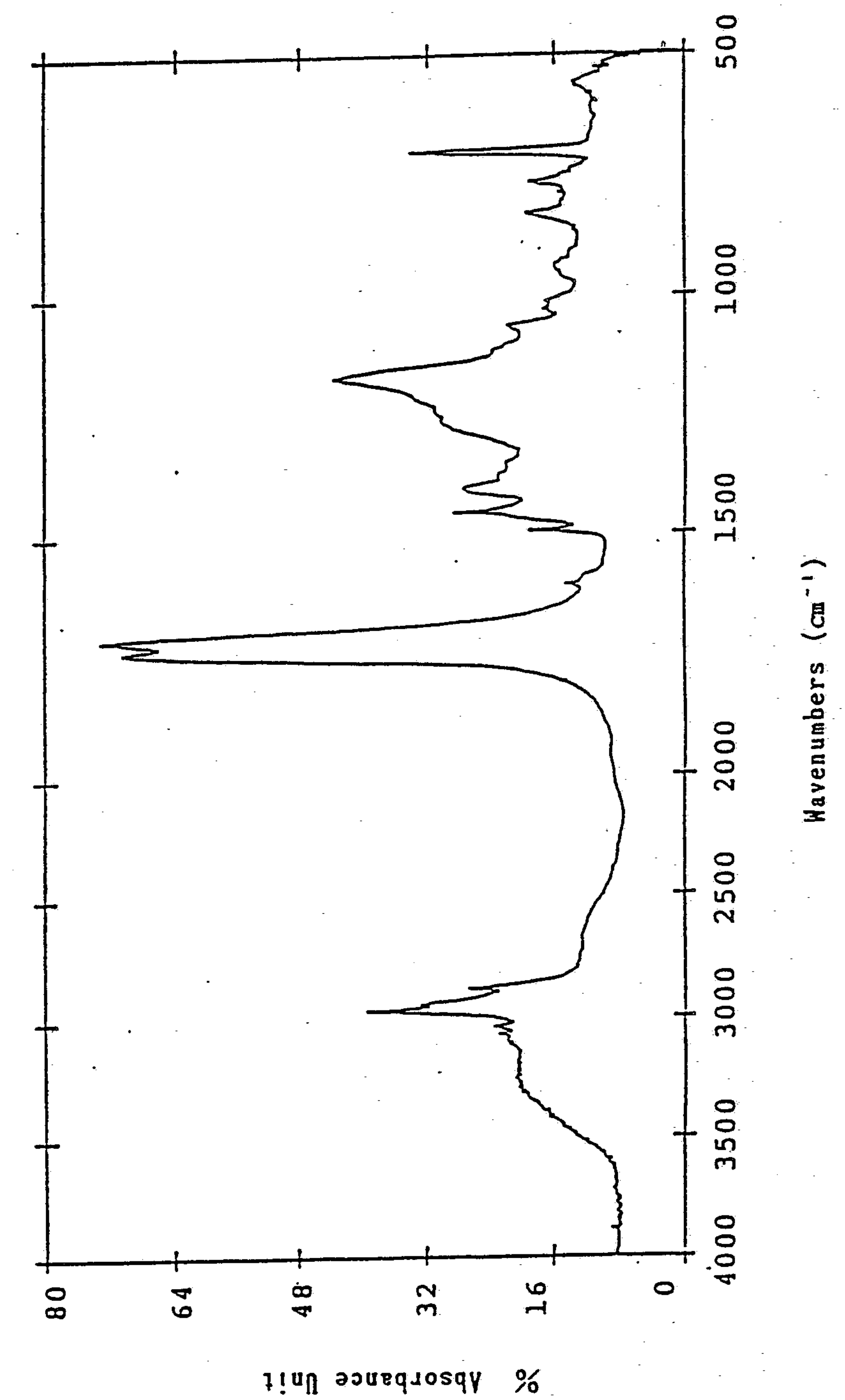
% Absorbance Unit
80
64
48
32
16
0
Wavenumbers (cm⁻¹)
4000
3500
3000
2500
2000
1500
1000
500

Figure 4

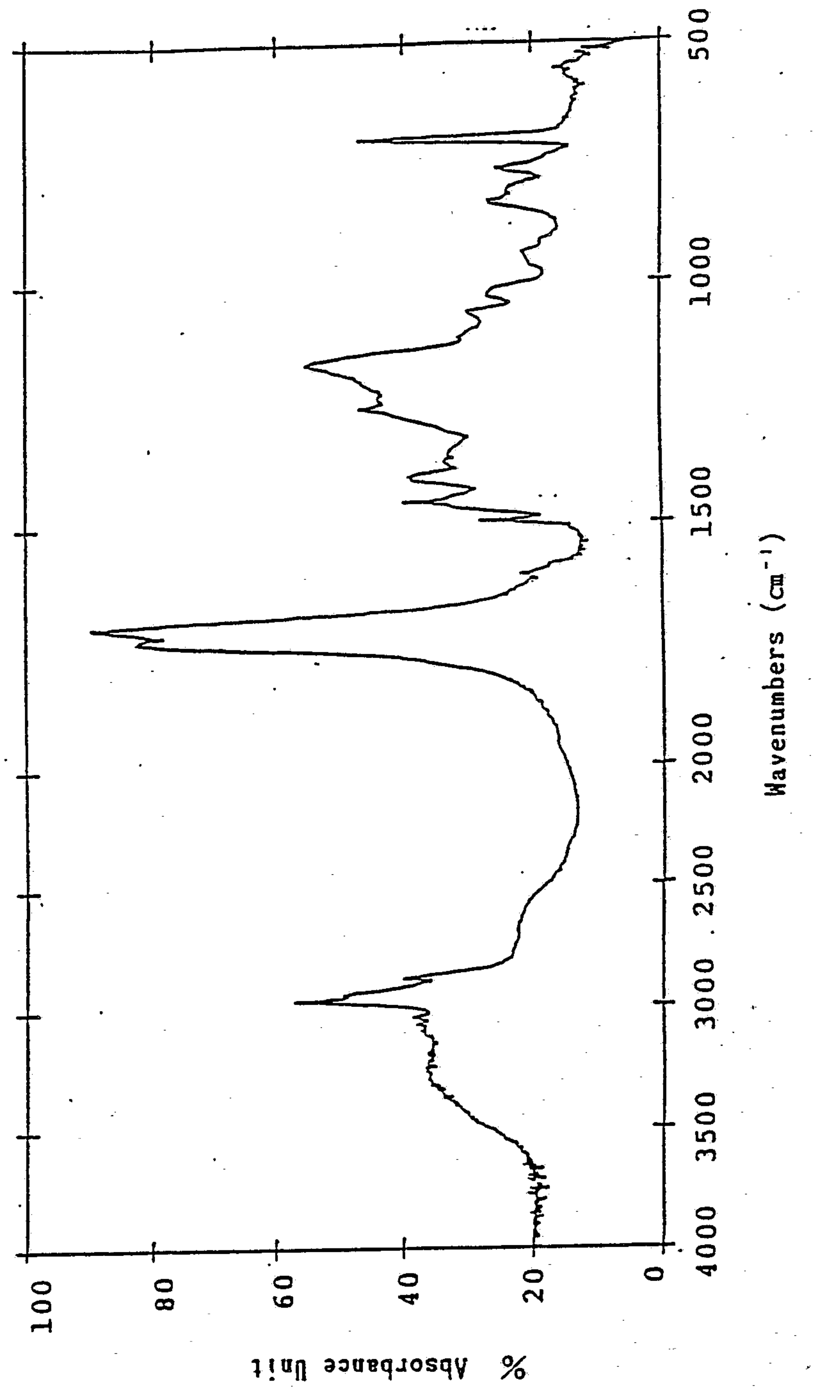
% Absorbance Unit
100
80
60
40
20
0
Wavenumbers (cm⁻¹)
4000
3500
3000
2500
2000
1500
1000
500

# Figure 5

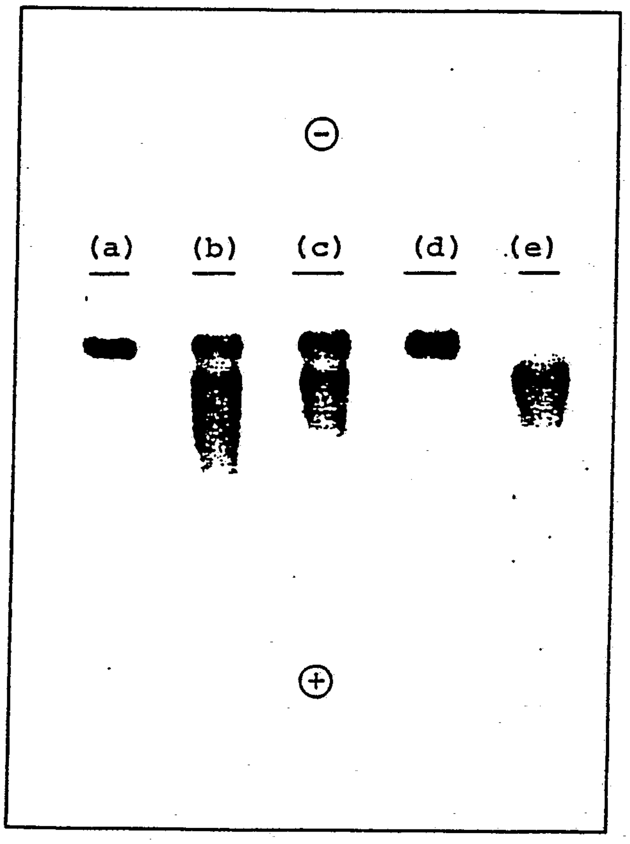

Figure 6

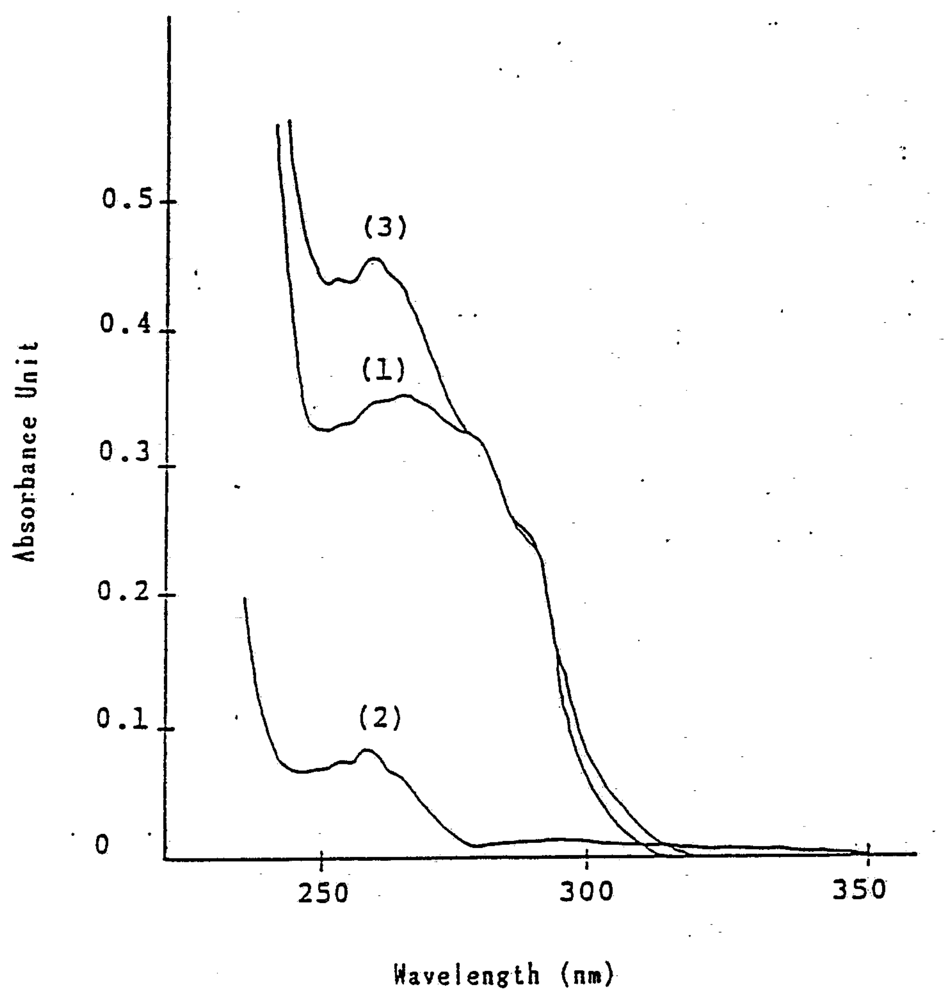

Figure 7

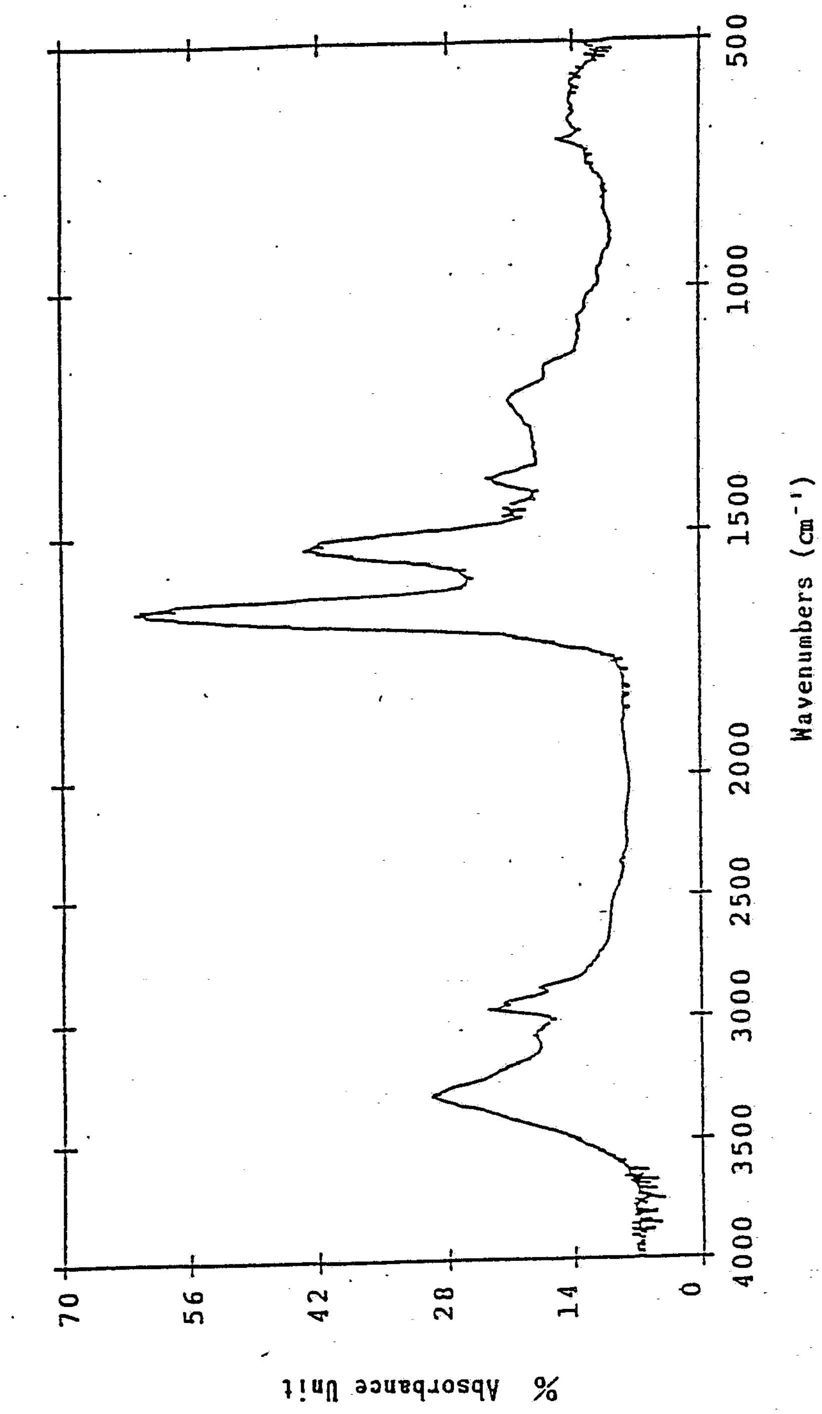
% Absorbance Unit
70
56
42
28
14
0
4000
3500
3000
2500
2000
1500
1000
500
Wavenumbers (cm-1)

# Figure 8

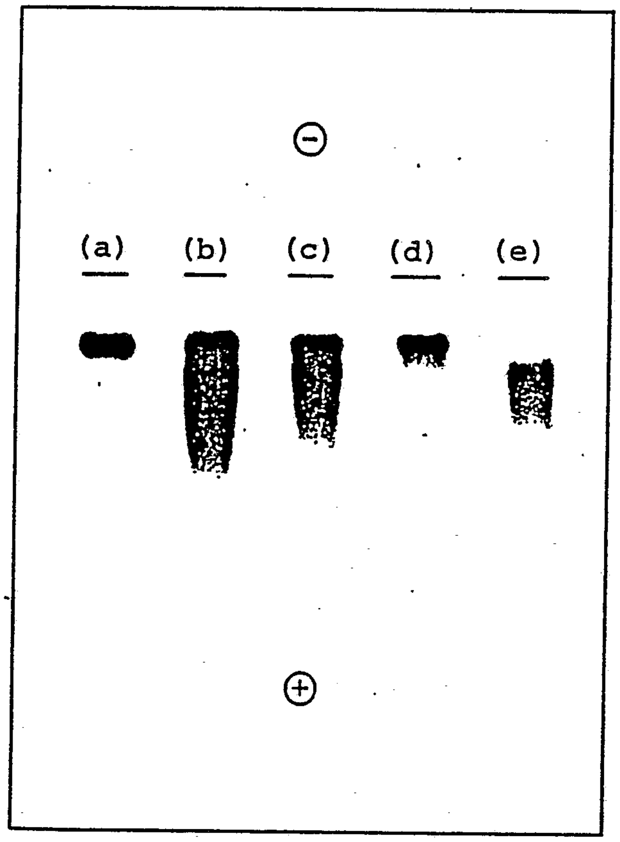

# Figure 9

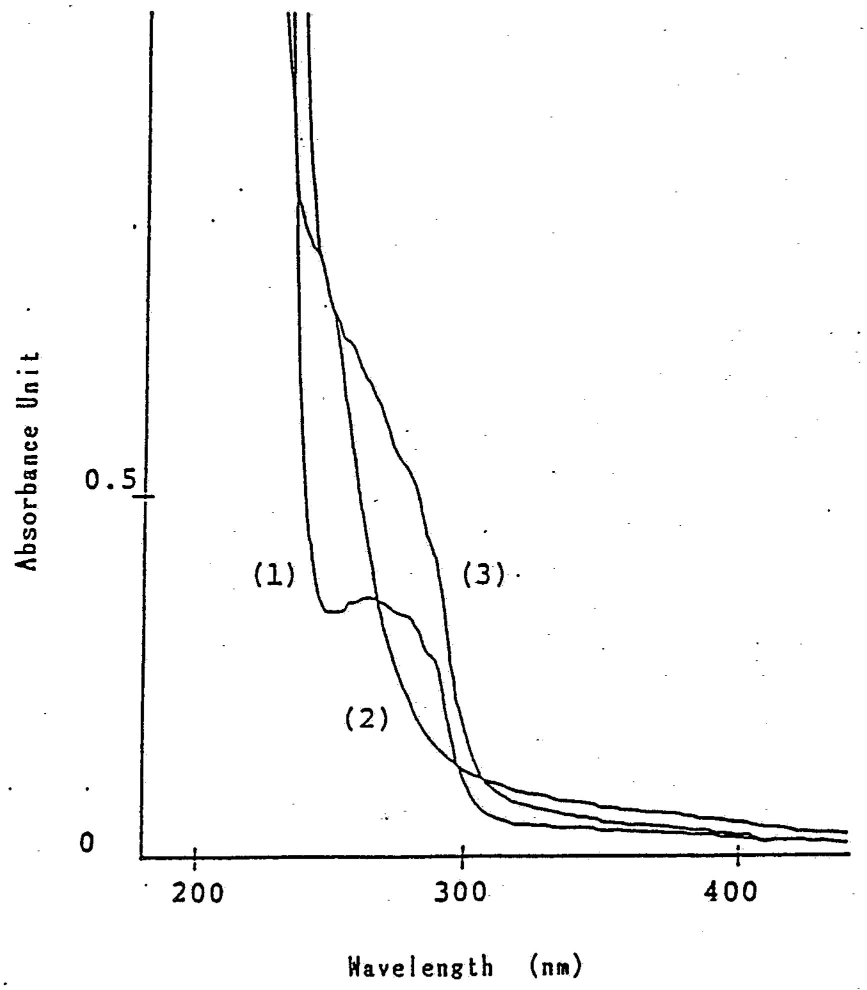

Figure 10

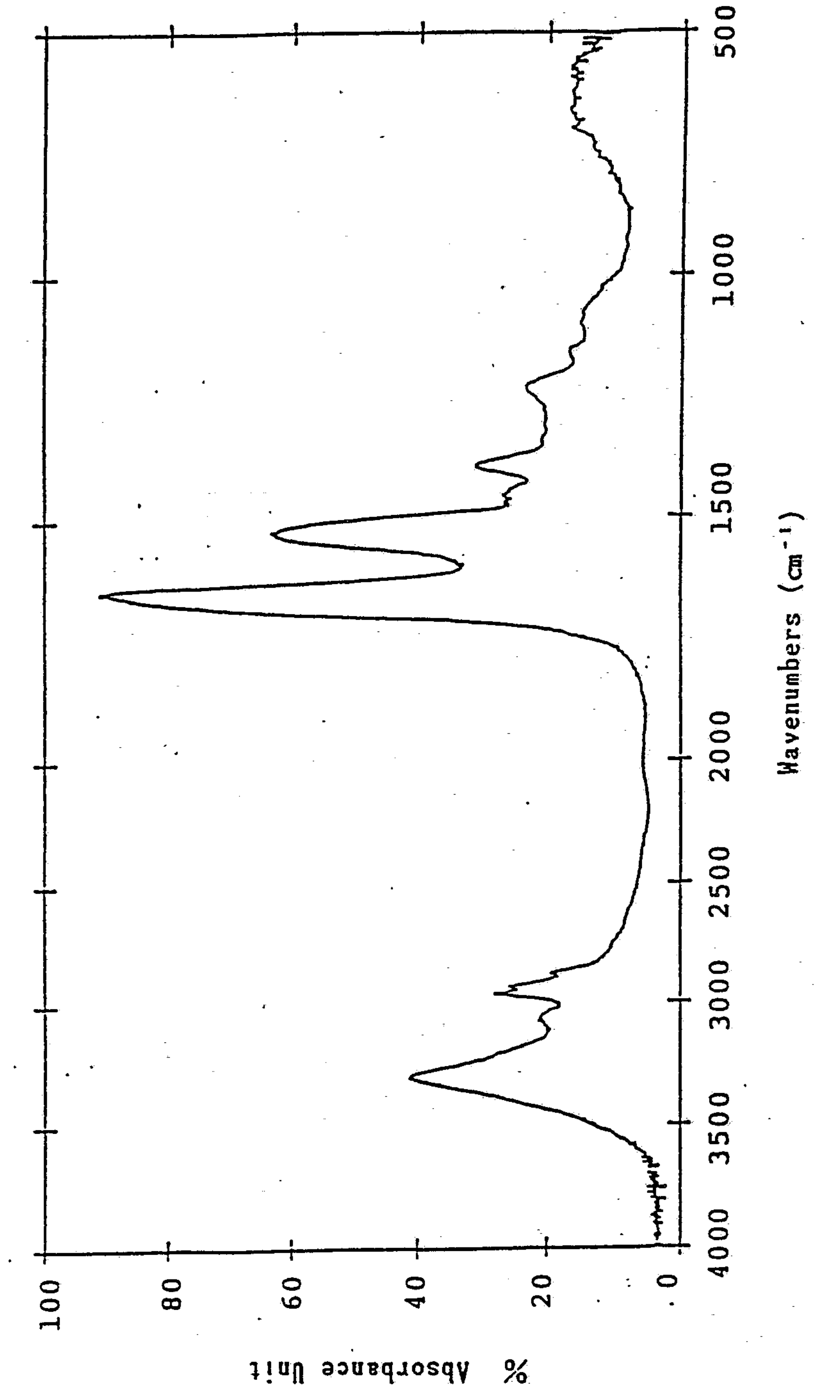
% Absorbance Unit
100
80
60
40
20
0
Wavenumbers (cm-1)
4000
3500
3000
2500
2000
1500
1000
500

Figure 11

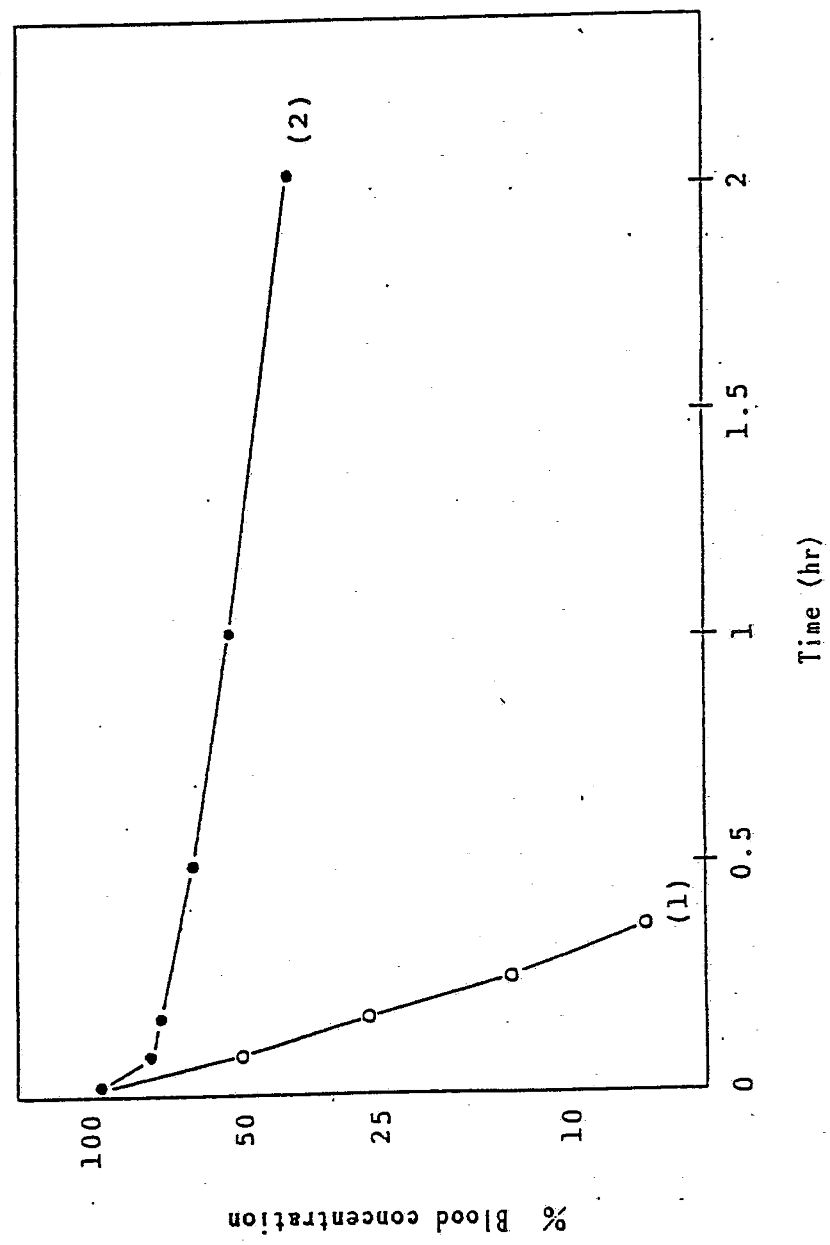

# Figure 12